(19) Europäisches Patentamt
European Patent Office
Office européen des brevets

(11) EP 4 219 410 A1

(12) # EUROPEAN PATENT APPLICATION

(43) Date of publication:
**02.08.2023 Bulletin 2023/31**

(21) Application number: **22382061.4**

(22) Date of filing: **27.01.2022**

(51) International Patent Classification (IPC):
***C02F 1/48*** *(2023.01)* ***G01N 1/34*** *(2006.01)*
***G01N 1/44*** *(2006.01)* ***G01N 5/04*** *(2006.01)*
***G01N 33/18*** *(2006.01)*

(52) Cooperative Patent Classification (CPC):
**G01N 1/34; C02F 1/001; C02F 1/302; C02F 1/488; G01N 1/44; G01N 5/04; G01N 33/18;** C02F 1/66; C02F 2103/007; C02F 2103/08; C02F 2103/22; C02F 2103/42; C02F 2305/026

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Captioplastic, S.L.**
  **28004 Madrid (ES)**
- **Universidad Autónoma de Madrid**
  **28049 Madrid (ES)**

(72) Inventors:
- **MUÑOZ GARCÍA, Macarena**
  **E-28049 Madrid (ES)**
- **ORTIZ SUÁREZ, David**
  **E-28049 Madrid (ES)**
- **NIETO-SANDOVAL RODRÍGUEZ, Julia**
  **E-28049 Madrid (ES)**
- **PARRA SÁNCHEZ, Raquel**
  **E-28004 Madrid (ES)**
- **MARTÍNEZ DE PEDRO, Zahara**
  **E-28049 Madrid (ES)**
- **CASAS DE PEDRO, José Antonio**
  **E-28049 Madrid (ES)**

(74) Representative: **ABG Intellectual Property Law, S.L.**
**Avenida de Burgos, 16D**
**Edificio Euromor**
**28036 Madrid (ES)**

(54) **METHOD FOR THE QUANTIFICATION AND ANALYSIS OF MICROPLASTICS PRESENT IN AQUEOUS MATRICES**

(57) The present invention relates to a process for the quantification and analysis of microplastics contained in aqueous matrices, comprising the magnetic separation of microplastics by the interaction of said microplastics with magnetic iron material particles, forming microplastic/magnetic iron material aggregates, the selective oxidation of the particles of a non-plastic organic nature, and the quantification and/ of the separated microplastics.

EP 4 219 410 A1

## Description

### Field of the Invention

The present invention relates to processes for determining parameters for the quantification of microplastics contained in aqueous matrices furthermore containing other non-plastic particles and fundamentally consisting of the magnetic separation of the microplastics from the aqueous matrix after interaction with magnetic iron material particles and formation of microplastic/magnetic iron material aggregates, the selective oxidation of organic (non-plastic) solid particles that could be separated together with the microplastics in the first step. The invention also relates to processes for the quantification of microplastics in said aqueous matrices using the previously determined parameters.

### Background of the Invention

The excellent properties of plastics, among which the easy molding, high mechanical and chemical resistance, and the low cost thereof stand out, have led to them being widely used both by industry and by society as a whole. Since the development of the first synthetic polymers in the mid 20$^{th}$ century, the production of these synthetic polymers has not stopped growing. In 2019, the global production of plastics reached 370 million tons (PlasticsEurope, 2020), and the manufacture of plastics is expected to double in the next twenty years (MacArthur, D.E. et al., The New Plastics Economy: Rethinking the Future of Plastics. World Economic Forum. (2016)). Although plastics are undeniably useful, the generation of plastic waste and the inadequate management thereof represent an important environmental problem.

The removal of plastic when said plastic is large-sized, is not problematic. However, plastic breaks up with use, generating small-sized pieces of plastic which are harder to remove, particularly when said pieces have a millimetric diameter. Microplastics, defined as plastic particles having a size less than 5 mm (Bergmann, M. et al., Marine Anthropogenic Litter (2015) Springer), are traces of plastic having a widespread presence in the environment. Microplastics have been found in practically all the ecosystems on Earth, including inland and marine water, sediments, air, and soils (Grbic, J. et al., Environmental Science and Technology Letters 6 (2019) 68-72). Accordingly, they are also present in organisms (Cole, M. et al., Nature Scientific Reports 4:4528 (2014) 1-8; Wieczorek, A.M. et al., Environmental Science and Technology 53 (2019) 5387-5396), fundamentally in aquatic organisms, so there is considerable concern for the potential damages that their presence may have both in humans and in fauna (Wright, S.L. et al., Environmental Science and Technology 51 (2017) 6634-6647; Cox, K.D. et al., Environmental Science and Technology 53 (2019) 7068-7074).

It is estimated that 80% of microplastics present in the ocean comes directly from rivers (Li, J. et al. Water Research 137 (2018) 362-374). In turn, rivers are the primary recipients of effluents from urban and industrial waste water treatment plants (WWTPs), which have been identified as important sources of introducing microplastics into the aquatic environment (Li, J. et al. Water Research 137 (2018) 362-374). In this aspect, the development of equipment which allows the treatment of microplastic-contaminated water represents an obviously relevant challenge.

On the other hand, to evaluate the true impact of microplastics and their distribution in the environment, as well as to develop a specific regulation in this field, it is essential to perform suitable quantification and characterization of these particles in the different aqueous matrices that may contain them (WWTP effluents, drinking water, rivers, lakes, reservoirs, and oceans, among others). Analysis of these particles is particularly complex given their highly heterogeneous size, shape, color, density, and chemical composition. In a recent review of the literature (Koelmans, A.A. et al. Water Research 155 (2019) 410-422), fifty papers that studied the presence of microplastics in inland waters and drinking water were analyzed. It was concluded that many of the papers could not be regarded as entirely reliable. The biggest challenge is with very small microplastics, i.e., those having a size less than 300 $\mu$m. For example, sampling with nets with a pore size of 80 $\mu$m instead of 330 $\mu$m gave rise to the determination of 250-fold higher concentrations (Dris, R. et al. Environmental Chemistry 12 (2015) 592-599). Considering the set of published studies about the presence of microplastics in inland waters, there is a clear need to develop an effective sampling system for the analysis of microplastics. In fact, the recent World Health Organization report entitled "Microplastics in drinking-water" (World Health Organization (2019) ISBN 978-92-4-151619-8) highlighted the development of methods for the sampling and analysis of microplastics as a needed priority in research.

The analysis of microplastics present in the aqueous matrices to be evaluated necessitates several steps (Prata, J.C. et al. Trends in Analytical Chemistry 110 (2019) 150-159). First, the microplastics must be extracted from the aqueous matrix. Second, the solid particles separated in the first step must be processed for the purpose of eliminating possible interferences, fundamentally organic particles. Third, and only in the event that the sample has a high content of non-plastic solids, a step of separating the microplastics may be needed. Lastly, the microplastics are quantified and characterized.

Filtration or sifting is by far the most common method for separating microplastics from aqueous matrices (Prata, J.C. et al. Trends in Analytical Chemistry 110 (2019) 150-159). The pore size of the filter or of the sieve used varies greatly and will logically determine the size of the microplastics that it will be able to separate out. Even still, very

small pore sizes are usually avoided since they lead to obstruction problems due to the presence of other solids in suspension in the water, and furthermore using very small pore sizes consumes a lot of time. Therefore, small pore sizes (0.45 - 100 μm) are normally only used in the analysis of particularly clean aqueous matrices such as groundwater and drinking water (Koelmans, A.A. et al. Water Research 155 (2019) 410-422). In that sense, Neston nets with a pore size of 333 μm, as well as plankton nets with a pore size of 100 μm, are most commonly used directly in sampling (Stock, F. et al. Trends in Analytical Chemistry 113 (2019) 84-92; Prata, J.C. et al. Trends in Analytical Chemistry 110 (2019) 150-159).

There are other less common methods for separating microplastics from aqueous matrices that furthermore allow the direct quantification thereof, such as electrostatic separators (Felising, S. et al. Environmental Pollution 234 (2018) 20-28) and flow cytometry (Sgier, L. et al. Nature Communication 7 (2016) 11587). Nevertheless, they require specialized equipment and long analysis times, in addition to entailing a high cost. The magnetic separation of microplastics due to their interaction with magnetic materials is a novel treatment that allows separating microplastics from water regardless of the size and nature thereof, which represents an important advantage with respect to conventional filtration methods (Grbic, J. et al., Environmental Science and Technology Letters, 6 (2019) 68-72; Munoz, M. et al., ES 2 796 998 A1 and WO 2020/240069 A1). Nevertheless, use thereof would involve an increase in the density of microplastics, which would hinder subsequent density separation from the rest of the solids present in the water and which would also have interacted with the magnetic material, such as non-plastic organic particles, for example. This may be why the magnetic separation of microplastics from water for quantification has not been investigated until now.

Aqueous matrices often contain solid particles in suspension, fundamentally of an organic nature. These particles can interfere in the analysis of microplastics, giving rise to an overestimation of their concentration, regardless of the extraction process used. Therefore, it is fundamental to use a treatment which allows significantly reducing the interference of non-plastic organic solid particles. This is a critical step for the analysis of these plastic particles. The most common methods for removing non-plastic solid particles in suspension are acid, alkaline, or enzymatic digestion or the application of oxidation processes. In acid digestion, nitric acid has shown the best results (Claessens, M. et al. Marine Pollution Bulletin 70 (2013) 227-233). Nevertheless, it is a fairly aggressive method that often gives rise to the partial dissolution of the microplastics (for example, polystyrene and polyethylene are very sensitive to acid treatment) and/or to the caking of said microplastics (Stock, F. et al. Trends in Analytical Chemistry 113 (2019) 84-92). Therefore, acid digestion is not widely used in the analysis of microplastics in aqueous matrices. Alkaline digestion has greater potential than acid digestion does because it is less aggressive for microplastics. Treatment with KOH (10% v/v) or with NaOH (10 M) at 60°C for 24 h has been the most effective (Hurley, R.R. et al. Environmental Science and Technology 52 (2018) 7409-7417). Nevertheless, some plastics such as polycarbonate, polystyrene, or polyethylene terephthalate also undergo degradation, with loss of mass, during treatment. In turn, enzymatic digestion emerges as an interesting alternative since it is less aggressive and less likely to cause damage to microplastics (Prata, J.C. et al. Trends in Analytical Chemistry 110 (2019) 150-159). However, enzymatic digestion efficacy will depend on the type of organic matter present in the sample. Therefore, use of this digestion often entails several steps, generally combined with gentle steps of oxidation using $H_2O_2$, and requires long processing times of up to two weeks (Löder, M.G. et al. Environ. Sci. Technol. 51 (2017) 14283-14292). Furthermore, it represents a high cost.

In this context, the use of oxidation processes represents a promising alternative for the removal of organic particles. The Fenton process in particular stands out given the simplicity of the necessary equipment, how fast it operates, and the low operating cost required. This process is based on the catalytic decomposition of $H_2O_2$ in the presence of iron salts in acid medium. This gives rise to the generation of hydroxyl radicals (HO·), species having a high oxidizing power and low selectivity capable of removing a wide range of organic compounds. A number of recent studies have proven the viability of this system in removing non-plastic organic solid particles in suspension and facilitating the isolation of the microplastics present in aqueous matrices of a different nature (Tagg, A.S. et al. Chem. Commun. 53 (2017) 372-375; Munno, K. et al. Environ. Toxicol. Chem. 37 (2018) 91-98; Hurley, R.R. et al. Environ. Sci. Technol. 52 (2018) 7409-7417; Cunsolo, S. et al. Anal. Bioanal. Chem. 413 (2021) 3789-3799). All the studies have been characterized by the use of high doses of $H_2O_2$ (hundreds of g/L) and of iron (1 - 30 g/L). The tested operating temperatures ranged from room temperature to a maximum of 60°C, because higher temperatures caused damages and losses of mass in the microplastics (Munno, K. et al. Environ. Toxicol. Chem. 37 (2018) 91-98). Accordingly, long reaction times, generally 24 h, have been required to achieve high degrees of removal of interfering organic particles. Another important limitation relates to the use of high iron concentrations (in the order of g/L), which invariably leads to the deposition iron precipitates on the surface of the microplastics. This clearly hinders the subsequent quantification thereof.

In view of this background, the development of an analysis protocol that allows quantifying microplastics in aqueous matrices which further comprise non-plastic organic particles, regardless of the composition and the size of the microplastics, in an efficient, fast, and cost-effective manner and is applicable to aqueous matrices of a different nature would represent a crucial finding in this field, where there is currently no standard method.

## Summary of the Invention

The inventors have surprisingly discovered that the use of a microwave-intensified Fenton process has proven to be very effective and fast for the oxidation of organic particles without affecting the entity of the microplastic particles. This process is extremely useful for processes for the quantification of microplastics.

As shown in the examples, yields both in the process of eliminating organic particles and in the process of quantifying microplastics are very high (greater than 80 and 95%, respectively) regardless of the nature or size of said microplastics, and also regardless of the characteristics of the aqueous matrix. The processes for the quantification of microplastics of the present invention are particularly advantageous with respect to other methods included in the state of the art since they entail a significant reduction in the time needed and in the consumption of reagents for achieving the quantification of microplastics in an aqueous matrix. Likewise, all the reagents used are environmentally friendly. An additional advantage of the processes of the present invention is the simplicity of the required equipment and the low cost of the materials and reagents used. Lastly, the methods have shown high reproducibility of the obtained results, which is a fundamental requirement for standardization.

Therefore, a first aspect the invention relates to a process for determining parameters for the quantification of microplastics in an aqueous matrix comprising microplastics and non-plastic organic particles, and optionally inorganic particles, the process comprising:

a1) providing a first sample of the aqueous matrix comprising microplastics, non-plastic organic particles, and optionally inorganic particles,
b1) adding magnetic iron material particles to the aqueous matrix provided in step a1) to form microplastic/magnetic iron material particle aggregates and to form non-plastic organic particle/magnetic iron material particle aggregates,
c1) separating the formed aggregates and the free magnetic material particles from the mixture of step b1) by applying a magnetic field,
d1) subjecting the aggregates and the free magnetic iron material particles separated in step c1) to a step of drying,
e1) determining the weight of dry solids of step d1), obtaining parameter A,
f1) preparing an aqueous suspension of the aggregates and the free magnetic iron material particles obtained in step d1),
g1) subjecting the aqueous suspension prepared in step f1) to a microwave-assisted Fenton process,
h1) separating the aggregates and the free magnetic material particles present in the aqueous suspension resulting from step g1), preferably by means of filtration,
i1) subjecting the aggregates and the free magnetic iron material particles separated in step h1) to a step of drying,
j1) determining the weight of dry solids in step i1), obtaining parameter AF,
k1) subjecting the aggregates and the free magnetic material particles separated in step i1) to a step of combustion, and
l1) determining the weight of solids obtained after step k1), obtaining parameter AFM.

In a second aspect, the invention relates to a process for determining parameters for the quantification of microplastics in an aqueous matrix comprising microplastics and non-plastic organic particles, and optionally inorganic particles, the process comprising:

a2) providing a first sample of the aqueous matrix comprising microplastics, non-plastic organic particles, and optionally inorganic particles,
b2) separating the solids from the sample of step a2), preferably by means of filtration,
c2) subjecting the solids separated in step b2) to a step of drying,
d2) determining the weight of dry solids of step c2), obtaining parameter $ST_M$,
e2) subjecting the dry solids of step d2) to a step of combustion, and
f2) determining the weight of solids obtained after step d2), obtaining parameter $SI_M$,
g2) providing a second sample of the aqueous matrix comprising microplastics, non-plastic organic particles, and optionally inorganic particles,
h2) adding magnetic iron material particles to the aqueous matrix provided in step g2) to form microplastic/magnetic iron material particle aggregates and to form non-plastic organic particle/magnetic iron material particle aggregates,
i2) separating the formed aggregates and the free magnetic material particles from the mixture of step h2) by applying a magnetic field,
j2) preparing an aqueous suspension of the aggregates and the free magnetic iron material particles separated in step i2),
k2) subjecting the aqueous suspension prepared in step j2) to a microwave-assisted Fenton process,
l2) separating the aggregates and the free magnetic material particles present in the aqueous suspension resulting

from step k2), preferably by means of filtration,
m2) subjecting the aggregates and the free magnetic iron material particles separated in step 12) to a step of drying,
n2) determining the weight of dry solids in step m2), obtaining parameter $STNOX_A$,
o2) subjecting the aggregates and the free magnetic material particles separated in step m2) to a step of combustion,
p2) determining the weight of solids obtained after step o2), obtaining parameter $SI_A$,
q2) filtering the residual aqueous fraction obtained after the separation of step i2),
r2) subjecting the solids separated in step q2) to a step of drying,
s2) determining the weight of dry solids of step r2), obtaining parameter $ST_{SN}$, and
t2) subjecting the dry solids of step r2) to a step of combustion,
u2) determining the weight of solids obtained after step t2), obtaining parameter $SI_{SN}$.

In a third aspect, the invention relates to a process for the quantification of microplastics in an aqueous matrix comprising microplastics and non-plastic organic particles, and optionally inorganic particles, the process comprising:

I-1) performing steps a1)-I1) of the process for determining parameters according to the first aspect for determining parameters A, AF, and AFM;
II-1) calculating the weight of microplastics ($MP_A$) by means of equation 1:

$$MP_A = A - AFM - MO_{NP,A} \qquad \text{Equation 1}$$

wherein

A is the weight of solids determined in step e1) of the process according to the first aspect,
**[0018]** AFM is the weight of solids determined in step I1) of according to the first aspect, $MO_{NP,A}$ is calculated by means of equation 2

$$MO_{NP,A} = (A - AF) * \frac{1}{a} \qquad \text{Equation 2}$$

wherein A is the weight of solids determined in step e1) of the process according to the first aspect, AF is the weight of solids determined in step j1) of the process according to the first aspect, and a is a number between 0.7 and 1, preferably between 0.8 and 0.9, most preferably 0.8.
In a fourth aspect, the invention relates to a process for the quantification of microplastics in an aqueous matrix comprising microplastics and non-plastic organic particles, and optionally inorganic particles, the process comprising:

I-2) performing steps a2)-u2) of the process for determining parameters according to the second aspect for determining parameters $ST_M$, $SI_M$, $STNOX_A$, $SI_A$, $ST_{SN}$, and $SI_{SN}$;
II-2) calculating the weight of microplastics ($MP_M$) by means of equation 3:

$$MP_M = (\%MP \cdot (SO_A))/100 \qquad \text{Equation 3}$$

wherein

%MP is calculated by means of equation 4

$$\%MP = \left(1 - \left(\frac{1}{RMPSO+1}\right)\right) \cdot 100 \qquad \text{Equation 4}$$

wherein RMPSO is calculated by means of equation 5

$$RMPSO = \left(1 - \left(\frac{PPSO}{a \cdot 100}\right)\right) / \left(\frac{PPSO}{a \cdot 100}\right) \qquad \text{Equation 5}$$

wherein a is a number between 0.7 and 1, preferably between 0.8 and 0.9, most preferably 0.8, and PPSO is calculated by means of equation 6

$$PPSO = \frac{(SO_A + SI_A - STNOX_A)}{SO_A} \cdot 100 \qquad \text{Equation 6}$$

wherein $STNOX_A$ is the weight of solids determined in step n2) of the process according to the second aspect, $SI_A$ is the weight of solids determined in step p2) of the process according to the second aspect, and $SO_A$ is calculated by means of equation 7

$$SO_A = \left( (SI_{SN} + SI_A - M) \cdot \frac{ST_M - SI_M}{SI_M} \right) - (ST_{SN} - SI_{SN}) \qquad \text{Equation 7}$$

wherein $SI_A$ is the weight of solids determined in step p2) of the process according to the second aspect, $SI_{SN}$ is the weight of solids determined in step u2) of the process according to the second aspect, $ST_{SN}$ is the weight of solids determined in step s2) of the process according to the second aspect, M is the weight of magnetic material particles added in step h2) of the process according to the second aspect, $ST_M$ is the weight of solids determined in step d2) of the process according to the second aspect, and $SI_M$ is the weight of solids determined in step f2) of the process according to the second aspect.

**Description of Figures**

Figure 1: Particle size distribution of the magnetic iron mineral (magnetite) used in the examples.

**Detailed Description of the Invention**

*Processes for determining parameters for the quantification of microplastics*

In the first aspect, the invention relates to a process for determining parameters for the quantification of microplastics in an aqueous matrix comprising microplastics and non-plastic organic particles, and optionally inorganic particles, the process comprising:

a1) providing a first sample of the aqueous matrix comprising microplastics, non-plastic organic particles, and optionally inorganic particles,
b1) adding magnetic iron material particles to the aqueous matrix provided in step a1) to form microplastic/magnetic iron material particle aggregates and to form non-plastic organic particle/magnetic iron material particle aggregates,
c1) separating the formed aggregates and the free magnetic material particles from the mixture of step b1) by applying a magnetic field,
d1) subjecting the aggregates and the free magnetic iron material particles separated in step c1) to a step of drying,
e1) determining the weight of dry solids of step d1), obtaining parameter A,
f1) preparing an aqueous suspension of the aggregates and the free magnetic iron material particles obtained in step d1),
g1) subjecting the aqueous suspension prepared in step f1) to a microwave-assisted Fenton process,
h1) separating the aggregates and the free magnetic material particles present in the aqueous suspension resulting from step g1), preferably by means of filtration,
i1) subjecting the aggregates and the free magnetic iron material particles separated in step h1) to a step of drying,
j1) determining the weight of dry solids in step i1), obtaining parameter AF,
k1) subjecting the aggregates and the free magnetic material particles separated in step i1) to a step of combustion, and
l1) determining the weight of solids obtained after step k1), obtaining parameter AFM.

In the second aspect, the invention relates to a process for determining parameters for the quantification of microplastics in an aqueous matrix comprising microplastics and non-plastic organic particles, and optionally inorganic particles, the process comprising:

a2) providing a first sample of the aqueous matrix comprising microplastics, non-plastic organic particles, and

optionally inorganic particles,
b2) separating the solids from the sample of step a2), preferably by means of filtration,
c2) subjecting the solids separated in step b2) to a step of drying,
d2) determining the weight of dry solids of step c2), obtaining parameter $ST_M$,
e2) subjecting the dry solids of step c2) to a step of combustion, and
f2) determining the weight of solids obtained after step e2), obtaining parameter $SI_M$,
g2) providing a second sample of the aqueous matrix comprising microplastics, non-plastic organic particles, and optionally inorganic particles,
h2) adding magnetic iron material particles to the aqueous matrix provided in step g2) to form microplastic/magnetic iron material particle aggregates and to form non-plastic organic particle/magnetic iron material particle aggregates,
i2) separating the formed aggregates and the free magnetic material particles from the mixture of step h2) by applying a magnetic field,
j2) preparing an aqueous suspension of the aggregates and the free magnetic iron material particles separated in step i2),
k2) subjecting the aqueous suspension prepared in step j2) to a microwave-assisted Fenton process,
l2) separating the aggregates and the free magnetic material particles present in the aqueous suspension resulting from step k2), preferably by means of filtration,
m2) subjecting the aggregates and the free magnetic iron material particles separated in step 12) to a step of drying,
n2) determining the weight of dry solids in step m2), obtaining parameter $STNOX_A$,
o2) subjecting the aggregates and the free magnetic material particles separated in step m2) to a step of combustion,
p2) determining the weight of solids obtained after step o2), obtaining parameter $SI_A$,
q2) filtering the residual aqueous fraction obtained after the separation of step i2),
r2) subjecting the solids separated in step q2) to a step of drying,
s2) determining the weight of dry solids of step r2), obtaining parameter $ST_{SN}$, and
t2) subjecting the dry solids of step r2) to a step of combustion,
u2) determining the weight of solids obtained after step t2), obtaining parameter $SI_{SN}$.

In the context of the present invention, the term "microplastic" or "microplastics" refers to a plastic material having an average diameter of less than 5 mm, preferably with an average diameter comprised in the range of 0.1 μm to 5 mm, more preferably in the range of 0.1 μm to 1 mm, even more preferably in the range of 0.1 μm to 500 μm, even more preferably from 100 μm to 250 μm. Measuring the size of microplastics involves the prior separation thereof from the aqueous matrix by means of vacuum filtration using a microporous membrane filter with a pore size of not more than 0.1 μm, and the subsequent drying thereof either at room temperature or in a drying oven at a temperature of not more than 100°C. The filter containing the microplastics is then analyzed by means of scanning electron microscopy, for example using the conditions described in the examples. The length and width of the microplastics are measured, and they are assigned the size of the largest dimension (length). The average diameter is defined as the average length of the microplastic particles (summation of lengths of the analyzed particles among the number of particles analyzed).

The microplastics can be formed by any plastic material, preferably synthetic organic polymers, such as polyethylene terephthalates (PET), polyethylenes (PE) including high-density polyethylenes (HDPE) and low-density polyethylenes (LDPE), polyvinyl chlorides (PVC), polypropylenes (PP), polystyrenes (PS), polycarbonates (PC), polyurethanes (PU), and combinations thereof.

"Polyethylene terephthalate" or "PET" is a polymer that can be obtained by means of a reaction of polycondensation between terephthalic acid and ethylene glycol. Polyethylene terephthalate has the following structure.

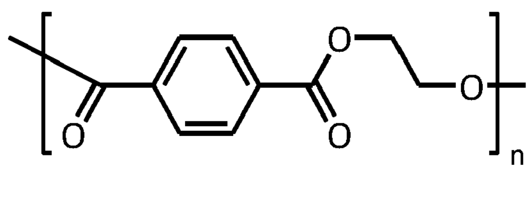

"Polyethylene" or "PE" refers to a polymer that can be obtained by the polymerization of ethylene. Polyethylene has the following structure.

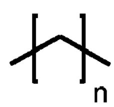

"High-density polyethylene" or "HDPE" refers to a polyethylene the density of which is comprised in the range of 0.930 to 0.970 g/mL. "Low-density polyethylene" or "LDPE" refers to a polyethylene the density of which is comprised in the range of 0.917 to 0.930 g/mL.

"Polyvinyl chloride" or "PVC" refers to a polymer that can be obtained by the polymerization of vinyl chloride. Polyvinyl chloride has the following structure.

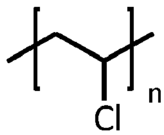

"Polypropylene" or "PP" refers to a polymer that can be obtained by the polymerization of propylene. Polypropylene has the following structure.

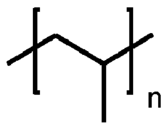

"Polystyrene" or "PS" refers to a polymer that can be obtained by the polymerization of styrene. Polystyrene has the following structure.

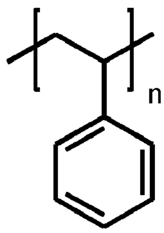

"Polycarbonate" or "PC" refers to a polymer comprising functional groups attached by carbonate groups (-O-C=O)-O-), such as for example diallyl diethylene glycol polycarbonate, or the most widely known, bisphenol A polycarbonate, the structure of which is shown below.

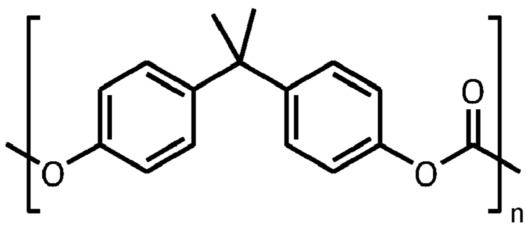

"Polyurethane" or "PU" refers to a polymer that can be obtained by the condensation of polyols, among which ethylene glycol and propylene glycol stand out, and polyisocyanates, among which methylene diphenyl diisocyanate (MDI) and toluene diisocyanate (TDI) stand out, giving rise to urethane groups (-NH-(C=O)-O-) in the structure of the polymer. There is a wide range of polyurethanes due to the range of polyisocyanates and polyols that can be used to form them.

In a preferred embodiment, the microplastic is selected from the group consisting of polystyrene, polyethylene terephthalate, polyethylene including high-density and low-density polyethylene, polyvinyl chloride, polypropylene, polycarbonate, polyurethane, and mixtures thereof; more preferably polystyrene, polypropylene, polyethylene terephthalate, polyethylene including high-density polyethylene and low-density polyethylene, and a mixture thereof; even more preferably, polystyrene, polypropylene, and a mixture thereof; most preferably polystyrene. An advantage of the process of the invention is its universal character because magnetic separation is effective regardless of the chemical nature of the microplastic.

The expression "aqueous matrix" refers to a composition containing water as its major component, preferably containing at least 80% by weight of water with respect to the total weight of the composition, more preferably at least 85%, even more preferably at least 90%, even more preferably at least 95%. The aqueous matrix may furthermore contain other substances that are already dissolved (for example, salts such as NaCl) or in suspension. Particularly, the aqueous matrix is selected from the group consisting of wastewater (including domestic, urban, industrial, agricultural, and livestock farming wastewater), sea water, river water, lake water, groundwater, tap water, or a mixture thereof. Therefore, wastewater purification plants, drinking water treatment plants, the agri-food industry (for example fish farms), sources of water for human consumption, water facilities for recreational purposes (for example, swimming pools), among others, are facilities of interest for the application of the process described in the present invention. The aqueous matrices comprise microplastics and further comprise non-plastic organic particles. Optionally, the aqueous matrices may further comprise inorganic particles. Therefore, in one embodiment the aqueous matrix comprises microplastics and non-plastic organic particles. In another embodiment, the aqueous matrix comprises microplastics, non-plastic organic particles, and inorganic particles. Preferably, the pH of the aqueous matrix must be between 1 and 13, more preferably between 2 and 12, more preferably between 3 and 10, more preferably between 5 and 9, even more preferably between 6 and 8, most preferably about 7. The pH of the aqueous matrix can be conditioned by adding a base, such as for example, sodium hydroxide, potassium hydroxide, or sodium carbonate, among others, or an acid, such as for example, nitric acid, hydrochloric acid, or sulfuric acid, among others, until reaching a pH value within the previously defined range. The pH can be determined by means of a pH-meter.

The process of the present invention allows the separation of microplastics regardless of the electrical conductivity of the water. It is effective both for waters with low levels of conductivity, less than 0.05 μS/cm, and for saline waters with high levels of conductivity, of 50 mS/cm. The term "electrical conductivity" refers to the capacity of any substance to allow an electric current to pass through same. Electrical conductivity can be determined by means of a conductivity meter.

Preferably, the concentration of solid particles in suspension in the aqueous matrix must be between 0.1 and 10000 mg/L, more preferably between 1 and 2000 mg/L, even more preferably between 10 and 100 mg/L. The total solid particles in suspension can be determined using processes known to one skilled in the art, such as for example gravimetric methods based on retaining solid particles in a glass fiber filter ("Standard methods for the examination of water and wastewater", "2540 Solids" (APHA, AWWA, WPCF, 1992); Standard UNE-EN 872:2006 Water quality. Determination of solids in suspension. Method by filtration through glass fiber filters).

Preferably, the solid particles in suspension present in the aqueous matrix will be determined as their amount and composition help to determine the amount of magnetic iron material, preferably magnetite, to be added in the processes of the invention and to carry out the microwave-assisted Fenton process of the processes of the invention.

The processes of the invention require providing one or more samples of an aqueous matrix comprising microplastics. The aqueous matrix also comprises non-plastic organic solid particles and may further comprise other solids as inorganic particles. In a particular embodiment of the processes of the invention, the aqueous matrix comprises inorganic particles. The term "sample" refers to an aliquot, fraction, or part of the aqueous matrix having the same qualitative and quantitative composition as the original aqueous matrix. The skilled person knows how to determine the suitable volume of aqueous matrix sample needed to carry out the processes of the invention. Preferably, the volume of the aqueous matrix samples provided in the processes of the invention is 10 mL to 10 L.

Steps b1) and h2) involve the incorporation of magnetic iron material particles to the aqueous matrix sample provided to form aggregates between the microplastics and the magnetic iron material particles. The magnetic iron material particles can also form aggregates with the non-plastic organic particles.

The term "magnetic iron material" refers to any natural or synthetic solid substance, comprising at least one iron species and having magnetic properties, i.e., it is attracted by a magnet. In a preferred embodiment, the magnetic iron material is inorganic. In a particular embodiment, the magnetic iron material is not coated, i.e., they are bare magnetic material particles. Preferably, the magnetic iron material has a magnetic saturation between 5 and 100 emu/g (between $5 \cdot 10^3$ and $10^5$ A/m), more preferably between 30 and 90 emu/g (between $30 \cdot 10^3$ and $90 \cdot 10^3$ A/m), even more preferably between 65 and 90 emu/g (between $65 \cdot 10^3$ and $90 \cdot 10^3$ A/m), most preferably between 70 and 90 emu/g (between $70 \cdot 10^3$ and $90 \cdot 10^3$ A/m). The magnetic properties of the magnetic iron material can be determined using a magnetometer with a SQUID (superconducting quantum interference device) sensor. The magnetization (M) of the magnetic iron material is measured depending on the external magnetic field applied (H) in the range of -10000 to 10000 Oe (which is equivalent to -797700 to 797700 A/m) at room temperature. The magnetic saturation value of the material is that value of M reached when any subsequent increase in H does not cause an increase in the magnetization of the material. Preferably, the iron material has an iron content of at least 10% by weight with respect to the total weight of the material, preferably at least 20% by weight, more preferably at least 25% by weight, even more preferably at least 30% by weight, even more preferably at least 35% by weight, even more preferably at least 40% by weight, even more preferably at least 45% by weight, even more preferably at least 50% by weight, even more preferably at least 55% by weight, even more preferably at least 60% by weight, even more preferably at least 65% by weight. The percentage of iron can be determined using

a total reflection x-ray fluorescence (TXRF) spectrometer using the method described in the examples.

Preferably, the magnetic iron material comprises an iron species selected from the group consisting of Fe(II), Fe(III), metallic Fe, and a mixture thereof. One example would be magnetic iron minerals, such as magnetite (formed by ferrous-diferric oxide $FeO \cdot Fe_2O_3$, sometimes formulated as $Fe_3O_4$), maghemite ($\gamma$-$Fe_2O_3$), ilmenite ($FeTiO_3$) and pyrrhotite ($Fe_{0.8-1}S$), among others. In a preferred embodiment, the magnetic iron material is the mineral magnetite.

The use of magnetic iron minerals, particularly magnetite, is advantageous from the economic and environmental viewpoint because they are materials that are abundant, environmentally-friendly, and readily separated from the aqueous matrices.

The magnetic iron material is in the form of particles. The term "particle" refers to the magnetic iron mineral being in the form of powder or granules with an average diameter of less than 5 mm, preferably the average diameter of which is comprised between 0.05 $\mu$m and 5 mm, more preferably between 0.05 $\mu$m and 1 mm, even more preferably between 0.05 $\mu$m and 500 $\mu$m, even more preferably between 0.05 $\mu$m and 100 $\mu$m, even more preferably between 0.1 $\mu$m and 10 $\mu$m, even more preferably between 0.1 $\mu$m and 5 $\mu$m, even more preferably between 0.1 $\mu$m and 1 $\mu$m, even more preferably between 0.1 $\mu$m and 0.5 $\mu$m, most preferably between 0.1 $\mu$m and 0.3 $\mu$m. Preferably, the particle size distribution must be sufficiently narrow in the established size range. The determination of the magnetic iron material particle size and distribution can be performed by means of scanning electron microscopy, for example, using the conditions described in the examples. The length and width of the microplastics are measured, and they are assigned the size of the largest dimension (length). The average diameter is defined as the average length (summation of lengths of the analyzed particles among the number of analyzed particles), with the length being the largest dimension of each magnetic iron material particle. For the determination of the particle size distribution, it is appropriate to use the statistical values D10, D50, and D90, which intercept 10%, 50%, and 90% by volume of the accumulated particles. In the preferred range, D10 (which indicates that 10% by volume of the particles of the sample have a diameter smaller than this value) is less than or equal to 0.3 $\mu$m, D50 (which indicates that 50% by volume of the particles of the sample have a diameter smaller than this value, and it represents the mean grain size) is less than or equal to 0.50 $\mu$m, and D90 (which indicates that 90% by volume of the particles of the sample have a diameter smaller than this value) is less than or equal to 0.8 $\mu$m. In the most preferred range, D10 (which indicates that 10% by volume of the particles of the sample have a diameter smaller than this value) is less than or equal to 0.15 $\mu$m, D50 (which indicates that 50% by volume of the particles of the sample have a diameter smaller than this value, and it represents the mean grain size) is less than or equal to 0.20 $\mu$m, and D90 (which indicates that 90% by volume of the particles of the sample have a diameter smaller than this value) is less than or equal to 0.35 $\mu$m. The magnetic iron material particles can be obtained by means of grinding the material and later sieving in the size ranges described above. The size of the magnetic iron material particles refers to the average diameter of said particles determined in dry conditions, without considering the agglomeration of the magnetic material particles when suspended in an aqueous matrix.

The terms "microplastic/magnetic iron material particle aggregates" or "microplastic/magnetic iron material aggregates" refer to a cluster of particles formed by the microplastics and the magnetic iron material particles preferably due to the interaction occurring between them. Likewise, the terms "organic particle/magnetic iron material particle aggregates" or "organic/magnetic iron material particle aggregates" refer to a cluster of particles formed by organic particles (non-microplastic) and the magnetic iron material particles due to the interaction occurring between them. Likewise, the terms "non-plastic organic particle/magnetic iron material particle aggregates" or "non-plastic organic/magnetic iron material particle aggregates" refer to a cluster of particles formed by non-plastic organic particles and the magnetic iron material particles due to the interaction occurring between them. This interaction is a physicochemical interaction; no change has been observed in the chemical structure, although there is an effective anchoring between the surface of both substances such that the aggregate remains intact despite the stirring or modification of the properties of the water (temperature, pH).

The expression "non-plastic organic particles" or "non-plastic organic solid particles" refers to particles which can have any shape and the average diameter of which is in the range of 0.1 $\mu$m to 1 cm, preferably from 0.1 $\mu$m to 5 mm, 0.1 $\mu$m to 5 mm, preferably from 0.1 $\mu$m to 1 mm, more preferably from 0.1 $\mu$m to 500 $\mu$m, even more preferably from 100 $\mu$m to 500 $\mu$m, even more preferably from 100 $\mu$m to 250 $\mu$m and which are not made of a plastic material, with the average diameter being the average length (summation of lengths of the analyzed particles among the number of analyzed particles) and the length being the largest dimension of each particle. Examples of plastics are the same as those described for microplastics. Examples of non-plastic (organic) material particles are of a plant origin (leaves, branches, seeds, fruits, etc.), an animal origin (bones, microcrustaceans, larvae, animal remains, etc.), a fungal origin, etc. The size of the particles can be determined the same way as described for microplastics.

The expression "inorganic particles" or "inorganic solid particles" refers to particles which can have any shape and the average diameter of which is in the range of 0.1 $\mu$m to 1 cm, preferably from 0.1 $\mu$m to 5 mm, 0.1 $\mu$m to 5 mm, preferably from 0.1 $\mu$m to 1 mm, more preferably from 0.1 $\mu$m to 500 $\mu$m, even more preferably from 100 $\mu$m to 500 $\mu$m, even more preferably from 100 $\mu$m to 250 $\mu$m, and of a mineral origin, with the average diameter being the average length (summation of lengths of the analyzed particles among the number of analyzed particles) and the length being

the largest dimension of each particle. Examples of inorganic particles are sands, clays, insoluble salts, metal oxides, etc. The size of the particles can be determined the same way as described for microplastics.

In a particular embodiment, the size of the non-plastic organic particles and of the inorganic particles is in the same range as the size of the microplastics, preferably all of them have an average diameter of 100 $\mu$m to 250 $\mu$m.

In a preferred embodiment, the amount of magnetic iron material particles (in a final concentration measured in the aqueous matrix itself), preferably magnetite, added to the aqueous matrix in step b1) and/or h2) is in the range of 1 mg/L to 4000 g/L, more preferably from 10 mg/L to 1000 g/L, even more preferably from 10 mg/L to 100 g/L, even more preferably from 10 mg/L to 10 g/L, most preferably from 100 mg/L to 1 g/L.

In a particular embodiment, the amount of magnetic iron mineral particles (by weight), preferably magnetite, added to the aqueous matrix in step b1) must be between 0.1 and 1000 times the amount of solid particles in suspension (by weight) present in the aqueous matrix sample provided in step a2) and/or g2), more preferably from 0.5 to 500 times, even more preferably from 1 to 100 times, even more preferably from 1 to 70 times, most preferably from 1 to 20 times.

To determine the amount of magnetic iron material to be added, an aliquot of the aqueous matrix can be taken and the amount of solid particles in suspension in the aqueous matrix determined. This involves the application of standardized methods for the determination of total solid particles in suspension known to one skilled in the art, such as for example gravimetric methods based on retaining solid particles in a glass fiber filter ("Standard methods for the examination of water and wastewater", "2540 Solids" (APHA, AWWA, WPCF, 1992); Standard UNE-EN 872:2006 Water quality. Determination of solids in suspension. Method by filtration through glass fiber filters). In general, in this method, a sample of a known volume of the aqueous matrix is filtered using an apparatus under a vacuum or low pressure through a glass fiber filter having a pore size of 0.45 $\mu$m; subsequently, the filter containing the solid particles that have been retained is dried at 105°C, and the mass of the retained solid is determined by means of a weigh difference with respect to the clean filter. The obtained result divided by the volume of filtered aqueous matrix allows obtaining the concentration of total solid particles in suspension. For determining the solid particles in suspension, the filter containing the solid particles that have been retained, dried at 105°C is subjected to burning at 550°C for at least one hour and until reaching a constant mass value. The final mass value, divided by the volume of filtered aqueous matrix, allows obtaining the concentration of inorganic solid particles in suspension. The concentration of organic solid particles in suspension (volatile solids) is determined by the difference between the concentration of the total solid particles in suspension and the inorganic solid particles in suspension (non-volatile solids).

All or part of the non-plastic organic particles present in the aqueous matrix form aggregates with the magnetic iron material. Preferably, at least 50% by weight of the non-plastic organic particles with respect to the total weight of non-plastic organic particles present in the aqueous matrix form aggregates with the magnetic iron material, more preferably at least 60%, even more preferably at least 70%, even more preferably at least 80%, even more preferably at least 85%, even more preferably at least 90%, even more preferably at least 95%, even more preferably at least 98%, even more preferably at least 99%, even more preferably 100%.

Preferably, at least 90% by weight of microplastics with respect to the total weight of microplastics present in the aqueous matrix form aggregates with the magnetic iron material, more preferably at least 95%, even more preferably at least 98%, even more preferably at least 99%, even more preferably 100%.

In a preferred embodiment, the mixture obtained in steps b1) and/or h2) is subjected to stirring before performing steps c1) and/ j2), respectively. Stirring favors the interaction of the magnetic iron material particles with the particles in suspension (including microplastics) present in the aqueous matrix, which gives rise to the formation of aggregates (microplastic/magnetic iron material and non-plastic organic particles/magnetic iron material). Stirring can be performed by means of any conventional method known to one skilled in the art, such as, for example, mechanical, magnetic, or electrostatic stirring, and more preferably, mechanical stirring.

Once aggregates are formed between the particles in suspension contained in the aqueous matrix (including microplastics and non-plastic organic particles) and the magnetic iron material, these aggregates are separated, together with the free magnetic iron material, from the aqueous matrix in steps c1) and/or i2), by applying a magnetic field.

Said magnetic field can be generated with a magnet or electromagnet, preferably by means of a magnet.

The processes of the present invention have the additional advantage of the simplicity of the equipment that is required.

In the process of the first aspect, the aggregates and the free magnetic iron material particles separated in step c1) are subjected to a step of drying. Said drying can be performed by means of any method that is suitable for removing the wastewater present and does not degrade the microplastics, preferably subjecting the separated solids (aggregates and free magnetic iron material particles) to a temperature of 100 to 200°C, preferably from 100 to 150°C, more preferably from 100 to 110°C, even more preferably about 105°C. The drying temperature is preferably maintained from 1 h to 3 h, more preferably from 1.5 h to 2.5 h.

Next, in the process of the first aspect, step e1) is performed for the determination of the weight of dry solids of step d1), thereby obtaining parameter A.

The next step of the processes of the invention, steps f1) and j2), relates to preparing an aqueous suspension

of the particle aggregates (both those of microplastics and of non-plastic organic particles) and the free magnetic iron material particles that have been previously separated and, in the case of the first aspect, also previously dried.

In a preferred embodiment, the volume of water used to resuspend the aggregates and the free magnetic iron material particles is in the range of 1 mL to 1000 mL, more preferably from 10 mL to 500 mL, even more preferably from 10 mL to 250 mL, most preferably from 10 mL to 100 mL.

The next step of the processes of the invention, steps g1) and k2), consists of selectively oxidizing the non-plastic organic particles (which would be in the form of aggregates with the magnetic iron material) that are previously separated without affecting the chemical and structural integrity of the microplastics present in the microplastic/magnetic iron material aggregates that are also separated.

In a preferred embodiment, the selective oxidation of the non-plastic organic particles (forming aggregates with the magnetic iron material) is performed by means of applying a microwave-assisted Fenton process.

In a preferred embodiment, the pH of the aqueous suspension of steps f1), g1), j2), and k2) is conditioned such that it is in the range of 2 to 5, more preferably between 2 and 4, more preferably between 2.5 and 3.5, most preferably about 3. The pH of the aqueous suspension can be adjusted by adding an acid, such as for example, nitric acids, hydrochloric acid, or sulfuric acid, among others, until reaching a pH value within the previously defined range. Preferably nitric acid is used. The pH can be determined by means of a pH-meter.

The selective oxidation of the non-plastic organic particles in the microwave-assisted Fenton process requires the addition of hydrogen peroxide and the salt of a transition metal such as Fe, Cu, Mn, and Co in the previous aqueous suspension. Preferably, the added salt from the previous group is an Fe salt. Preferably, an iron salt selected from the group consisting of iron nitrate, iron chloride, iron sulfate, and a mixture thereof, preferably iron nitrate, is used as a precursor of iron in solution.

In a preferred embodiment, the amount of iron added is the amount needed to provide a final dissolved iron concentration in the aqueous suspension itself of step e); it is in the range of 1 mg/L to 1000 mg/L, more preferably from 1 mg/L to 100 mg/L, even more preferably from 5 mg/L to 100 mg/L, most preferably from 10 mg/L to 100 mg/L.

In a preferred embodiment, the amount of dissolved iron (by weight) added to the aqueous suspension of steps g1) and k2) is 0.01 to 5 times the amount of organic particles (by weight), including microplastics and non-plastic organic particles as defined above, present in the aqueous matrix sample provided in steps a1) and g2), respectively, more preferably from 0.1 to 2 times, even more preferably from 0.5 to 2 times, most preferably from 0.5 to 2 times. To determine the amount of dissolved iron to be added, an aliquot of the aqueous matrix can be taken and the amount of solid particles in suspension in the aqueous matrix determined. This involves the application of standardized methods for the determination of total solid particles in suspension known to one skilled in the art, such as for example gravimetric methods based on retaining solid particles in a glass fiber filter ("Standard methods for the examination of water and wastewater", "2540 Solids" (APHA, AWWA, WPCF, 1992); Standard UNE-EN 872:2006 Water quality. Determination of solids in suspension. Method by filtration through glass fiber filters). In general, in this method, a sample of a known volume of the aqueous matrix is filtered using an apparatus under a vacuum or low pressure through a glass fiber filter having a pore size of 0.45 $\mu$m; subsequently, the filter containing the solid particles that have been retained is dried at 105°C, and the mass of the retained solid is determined by means of a weight difference with respect to the clean filter. The obtained result divided by the volume of filtered aqueous matrix allows obtaining the concentration of total solid particles in suspension. For determining the inorganic and organic solid particles in suspension, the filter containing the solid particles that have been retained, dried at 105°C is subjected to burning at 550°C for at least one hour and until reaching a constant mass value. The final mass value, divided by the volume of filtered aqueous matrix, allows obtaining the concentration of inorganic solid particles in suspension. The concentration of organic solid particles in suspension (volatile solids) is determined by the difference between the concentration of total solid particles in suspension and inorganic solid particles in suspension (non-volatile or fixed solids).

In a preferred embodiment, the amount of hydrogen peroxide added to the aqueous suspension of steps g1) and k2) is the amount needed to provide a final concentration measured in the aqueous suspension itself which is in the range of 10 mg/L to 100 g/L, more preferably from 10 mg/L to 50 g/L, even more preferably from 100 mg/L to 50 g/L, even more preferably from 100 mg/L to 25 g/L, even more preferably from 1 g/L to 25 g/L, most preferably from 1 g/L to 10 g/L.

In a preferred embodiment, the amount of hydrogen peroxide (by weight) added to the aqueous suspension of steps g1) and k2) is 1 to 10000 times the amount of organic solid particles (by weight), including microplastics and non-plastic organic particles present in the aqueous matrix sample provided in steps a1 and g2), respectively, more preferably from 10 to 1000 times, even more preferably from 100 to 1000, most preferably from 100 to 500. To determine the amount of hydrogen peroxide to be added, an aliquot of the aqueous matrix can be taken and the amount of organic particles, including microplastics and non-plastic organic particles in the aqueous matrix determined. This involves the application of standardized methods for the determination of total solid particles in suspension known to one skilled in the art, such as for example gravimetric methods based on retaining solid particles in a glass fiber filter ("Standard methods for the examination of water and wastewater", "2540 Solids" (APHA, AWWA, WPCF, 1992); Standard UNE-EN 872:2006 Water quality. Determination of solids in suspension. Method by filtration through glass fiber filters). In general, in this method, a sample of a known volume of the aqueous matrix is filtered using an apparatus under a vacuum or low pressure through a glass fiber filter having a pore size of 0.45 μm; subsequently, the filter containing the solid particles that have been retained is dried at 105°C, and the mass of the retained solid is determined by means of a weight difference with respect to the clean filter. The obtained result divided by the volume of filtered aqueous matrix allows obtaining the concentration of total solid particles in suspension. For determining the inorganic and organic particles in suspension including microplastics and non-plastic organic particles, the filter containing the solid particles that have been retained, dried at 105°C is subjected to burning at 550°C for at least one hour and until reaching a constant mass value. The final mass value, divided by the volume of filtered aqueous matrix, allows obtaining the concentration of inorganic solid particles. The concentration of organic particles is determined by the difference between the concentration of total particles in suspension and inorganic particles in suspension.

In a particular embodiment, both the iron salt and the hydrogen peroxide are added in a single addition at the start of the oxidation reaction of steps g1) and k2).

Preferably, the process of the invention is performed by adding the iron salt and the hydrogen peroxide in several consecutive cycles during steps g1) and k2) to favor the reaction.

Every time the iron and hydrogen peroxide reagents are added to the aqueous suspension during steps g1) and k2), the aqueous suspension is introduced in a microwave to carry out the selective oxidation reaction.

In a preferred embodiment, the microwave power is in the range of 50 to 1500 W, more preferably from 500 to 1200 W, even more preferably from 600 to 1100 W, most preferably from 700 to 1000 W.

In a preferred embodiment, the reaction time for carrying out the selective oxidation of non-plastic organic solid particles by means of microwave-assisted Fenton of steps g1) and k2) is in the range from 1 s to 2 h, more preferably from 1 s to 1 h, even more preferably from 1 s to 30 min, even more preferably from 1 s to 10 min, most preferably from 2 min to 5 min. Reaction times refer to the total oxidation time, which oxidation can be performed either by means of two or more shorter cycles, for example, 2, 3, 4, 5, or 6 cycles, or all in one shot.

In a preferred embodiment, the temperature of the sample during the microwave-enhanced Fenton reaction reaches at least 80°C, preferably from 80 to 120°C, more preferably from 90 to 100°C.

The next step of the processes of the invention, steps h1) and 12), is the separation of the aggregates and the free magnetic material particles present in the aqueous suspension resulting from steps g1) and k2), respectively, preferably by means of filtration, more preferably by means of vacuum filtration.

The next step of the processes of the invention, steps i1) and m2), is to subject the aggregates and the free magnetic iron material particles separated in steps h1) and 12), respectively, to a step of drying. Said drying can be performed by means of any method that is suitable for removing the wastewater present, preferably subjecting the separated solids to a temperature of 100 to 200°C, preferably from 100 to 150°C, more preferably from 100 to 110°C, even more preferably about 105°C. The drying temperature is preferably maintained from 1 h to 3 h, more preferably from 1.5 h to 2.5 h.

Next, in the process of the first aspect, step j1) is performed for the determination of the weight of dry solids of step i1), thereby obtaining parameter AF, whereas step n2) is performed in the process of the second aspect for the determination of the weight of dry solids of step m2), thereby obtaining parameter $STNOX_A$.

Next, in the processes of the invention, in steps k1) and o2), the aggregates and the free magnetic material particles separated in steps i1) and m2), respectively, are subjected to a step of combustion. Preferably, the combustion is performed in an air atmosphere. Preferably, the combustion is performed by means of treatment at a temperature of 400 to 600°C, more preferably from 500 to 600°C, even more preferably about 550°C. In particular, this treatment lasts from 1 to 4 hours, preferably from 1 to 3 hours, more preferably from 1.5 to 2.5 h, most preferably about 2 h. This treatment assures the combustion of any plastic material present in the sample. This treatment would not affect inorganic particles which may optionally be present, or the magnetic iron material. The step of combustion removes the organic particles present in the aggregates.

Next, in the processes of the invention, step l1) or p2) is performed for the determination of the weight of solid particles obtained after steps of combustion k1) and o2), respectively. Therefore, in the process according to the first aspect of the invention, parameter AFM is obtained, whereas in the process according to the second aspect of the invention, parameter $SI_A$ is obtained.

All the determinations of the weight of solids are preferably performed once the corresponding solid has cooled to 10-35°C, more preferably to 20-25°C.

The second aspect of the invention further comprises additional steps a2)-f2) and steps q2)-u2).

Steps a2)-f2) are performed on a sample of the aqueous matrix comprising microplastics, non-plastic organic particles, and optionally inorganic particles different from the sample used in steps g2)-p2) described above, but both samples are from the same aqueous matrix.

Step a2) refers to providing a sample of the aqueous matrix comprising microplastics, non-plastic organic particles, and optionally inorganic particles. Said step is as defined above for steps a1) and g2).

Step b2) refers to the separation of solids from the sample of step a2), preferably by means of filtration, more preferably by means of vacuum filtration.

Step c2) refers to subjecting the solids separated in step b2) to a step of drying. The step of drying is as defined above for steps d1), i1), and m2).

Step d2) refers to the determination of the weight of dry solids of step c2), obtaining parameter $ST_M$.

Step e2) refers to subjecting the dry solids of step c2) to a step of combustion. The step of combustion is as defined above for steps k1) and o2).

Step f2) refers to the determination of the weight of solids obtained after step e2), obtaining parameter $SI_M$.

Steps g2)-u2) are performed on the same sample of the aqueous matrix comprising microplastics, non-plastic organic particles, and optionally inorganic particles used in steps g2)-p2) described above; they are a continuation of the treatment of said sample described in steps g2)-p2).

Step q2) refers to the filtration of the residual aqueous fraction obtained after the separation of step i2), preferably vacuum filtration.

Step r2) refers to subjecting the solids separated in step q2) to a step of drying. The step of drying is as defined above for steps d1), i1), and m2).

Step s2) relates to the determination of the weight of dry solids of step r2), obtaining parameter $ST_{SN}$.

Step t2) refers to subjecting the dry solids of step r2) to a step of combustion. The step of combustion is as defined above for steps k1) and o2).

Step u2) refers to the determination of the weight of solids obtained after step t2), obtaining parameter $SI_{SN}$.

*Processes for the quantification of microplastics*

In a third aspect, the invention relates to a process for the quantification of microplastics in an aqueous matrix comprising microplastics and non-plastic organic particles, and optionally inorganic particles, the process comprising:

I-1) performing steps a1)-l1) of the process for determining parameters according to the first aspect for determining parameters A, AF, and AFM;

II-1) calculating the weight of microplastics ($MP_A$) by means of equation 1:

$$MP_A = A - AFM - MO_{NP,A} \quad \text{Equation 1}$$

wherein

A is the weight of solids determined in step e1) of the process according to the first aspect,

AFM is the weight of solids determined in step l1) of according to the first aspect, $MO_{NP,A}$ is calculated by means of equation 2

$$MO_{NP,A} = (A - AF) * \frac{1}{a} \qquad \text{Equation 2}$$

wherein A is the weight of solids determined in step e1) of the process according to the first aspect, AF is the weight of solids determined in step j1) of the process according to the first aspect, and a is a number between 0.7 and 1, preferably between 0.8 and 0.9, most preferably 0.8.

The process of the third aspect comprises a first step 1-1 for determining parameters relevant to the quantification of microplastics. This step involves performing the process defined in the first aspect.

A, AFM, and AF have to be expressed in the same units of weight.

The process of the third aspect also comprises a second step 11-1 of calculating the weight of microplastics. This step is performed using equations 1 and 2. Nevertheless, this process is not limited to said equations but rather encompasses any mathematically equivalent equation or mathematical combination of said equations.

In a fourth aspect, the invention relates to a process for the quantification of microplastics in an aqueous matrix comprising microplastics and non-plastic organic particles, and optionally inorganic particles, the process comprising:

I-2) performing steps a2)-u2) of the process for determining parameters according to the second aspect for determining parameters $ST_M$, $SI_M$, $STNOX_A$, $SI_A$, $ST_{SN}$, and $SI_{SN}$;

II-2) calculating the weight of microplastics ($MP_M$) by means of equation 3:

$$MP_M = (\%MP \cdot (SO_A))/100 \qquad \text{Equation 3}$$

wherein

%MP is calculated by means of equation 4

$$\%MP = \left(1 - \left(\frac{1}{RMPSO+1}\right)\right) \cdot 100 \qquad \text{Equation 4}$$

wherein RMPSO is calculated by means of equation 5

$$RMPSO = \left(1 - \left(\frac{PPSO}{a \cdot 100}\right)\right) / \left(\frac{PPSO}{a \cdot 100}\right) \qquad \text{Equation 5}$$

wherein a is a number between 0.7 and 1, preferably between 0.8 and 0.9, most preferably 0.8, and PPSO is calculated by means of equation 6

$$PPSO = \frac{(SO_A + SI_A - STNOX_A)}{SO_A} \cdot 100 \qquad \text{Equation 6}$$

wherein $STNOX_A$ is the weight of solids determined in step n2) of the process according to the second aspect, $SI_A$ is the weight of solids determined in step p2) of the process according to the second aspect, and $SO_A$ is calculated by means of equation 7

$$SO_A = \left((SI_{SN} + SI_A - M) \cdot \frac{ST_M - SI_M}{SI_M}\right) - (ST_{SN} - SI_{SN}) \qquad \text{Equation 7}$$

wherein $SI_A$ is the weight of solids determined in step p2) of the process according to the second aspect, $SI_{SN}$ is the weight of solids determined in step u2) of the process according to the second aspect, $ST_{SN}$ is the weight of solids determined in step s2) of the process according to the second aspect, M is the weight of magnetic material particles added in step h2) of the process according to the second aspect, $ST_M$ is the weight of solids determined in step d2) of the process according to the second aspect, and $SI_M$ is the weight of solids determined in step f2) of the process according to the second aspect.

The process of the fourth aspect comprises a first step 1-2 of determining parameters relevant to the quantification of microplastics. This step involves performing the process defined in the second aspect.

Parameters $ST_M$, $SI_M$, $STNOX_A$, $SI_A$, $ST_{SN}$, and $SI_{SN}$ have to be expressed in the same units of weight.

The process of the fourth aspect also comprises a second step 11-2 of calculating the weight of microplastics. This step is performed using equations 3-7. Nevertheless, this process is not limited to said equations but rather encompasses any mathematically equivalent equation or mathematical combination of said equations.

Illustrative examples disclosing the features and advantages of the invention are described below; however, they must not be interpreted as being limiting of the object of the invention as defined in the claims.

**Examples**

***Materials and methods***

***Determination of the microplastic particle size and shape***

In the present invention, the microplastics were added to the aqueous matrices to subsequently carry out their magnetic separation by means of the process described in the specification. The particle size range of the microplastics was previously determined by means of sieving same using sieves of different sizes (5 mm, 1 mm, 500 μm, 250 μm, 100 μm, 50 μm, and 20 μm).

Measuring the size of microplastics in real aqueous matrices involves the prior separation thereof from the aqueous matrix by means of vacuum filtration using a microporous membrane filter with a pore size of not more than 0.1 μm, and the subsequent drying thereof either at room temperature or in a drying oven at a temperature of not more than 100°C. The filter containing the microplastics is then analyzed by means of light microscopy. In this case, a Nikon Eclipse Ci-S/Ci-L upright light microscope equipped with a DS-Fi2 digital camera and a DS-U3 control unit, with software for measuring and processing images (NIS-Elements L imaging) was used. A JEOL JSM 6335F scanning electron microscope, with 10X to 500,000X amplification was also used. The length and width of the microplastics are measured, and they are assigned the size of the largest dimension (length). The shape of the microplastics is analyzed by observing images of the particles at different magnifications.

***Determination of the composition of the microplastics***

The composition of the microplastics was determined by means of Fourier transform infrared spectroscopy (FTIR). The samples of microplastics were analyzed following the conventional analysis method using KBr pellets. The pellets were prepared by adding 1 mg of a sample of microplastic and about 99 mg of KBr. The obtained powder mixture was compacted by applying 10 tons of pressure by means of a hydraulic press, obtaining the KBr pellets. The samples were then analyzed by means of the Spectrum Two spectrophotometer (Perkin Elmer). Transmission tests were performed in the spectral range of 4000 - 450 $cm^{-1}$. The obtained spectra were compared with library databases to determine the type of polymer that forms the microplastic using Perkin Elmer Spectrum Software.

***Determination of the magnetic iron material particle size***

The magnetic iron material particle size (average diameter) and particle distribution were determined by means of the scanning electron microscopy analysis of the dry material. The equipment used was a JEOL JSM 6335F, with 10X to 500,000X amplification. The length and width of the magnetic iron material particles are measured, and they are assigned the size of the largest dimension (length). For the determination of the particle size distribution, it is appropriate to use the statistical values D10, D50, and D90, which intercept 10%, 50%, and 90% by volume of the accumulated particles.

***Determination of the percentage of iron***

The iron content of the magnetic iron material was determined using a total reflection x-ray fluorescence (TXRF) spectrometer, namely the S2 Peakfox (Bruker) model, with a molybdenum source. The method of preparation of the sample consisted of the open vessel digestion thereof using a solution of hydrochloric acid, nitric acid, and hydrofluoric acid at a proportion by volume of 6:2:0.5, at 105°C for 1.5 h. The concentration of material in the vessel was 1000 mg/L and a total volume of 10 mL was used. If necessary, the magnetic iron material was previously ground to a size less than 100 μm. Once the material sample was digested, an internal gallium standard was added in the solution for later quantification. A 3-μL aliquot of the resulting solution was introduced in the spectrometer for analysis, which was performed at 50 kV and 600 μA with an acquisition time of 500 s. The integration of the peak corresponding to the iron in the obtained spectrogram allowed determining the percentage of iron by weight contained in the material.

***X-ray diffraction***

Powder x-ray diffraction (PXRD) was used to perform the identification and characterization of the crystalline phases present in the magnetic iron material. The samples were analyzed in an X'Pert PRO theta/theta diffractometer by Panalytical with a Cu tube (K$\alpha$ radiation, 8.04 keV). The diffractometer has a secondary graphite monochromator and a Xe gas detector. A theta/2theta scan was performed with a 2$\theta$ range of 10 to 80°, an angular increment of 0.04°, and an accumulation time of 4s. Prior to analysis, the magnetic iron material particles were ground, if necessary, to a size smaller than 100 μm. For the identification of the crystalline phases present in the obtained diffractograms, the High Score Plus software and the ICDD (International Centre for Diffraction Data) PDF-4 Full File crystallographic database were used

***Determination of magnetic saturation***

The magnetic properties of the magnetic iron material were measured using a Quantum Design MPMS XL-5 magnetometer with a SQUID (superconducting quantum interference devices) sensor. If necessary, the magnetic iron material was previously ground to a size smaller than 100 μm. The magnetic moment (M) of the magnetic iron material was measured depending on the applied external magnetizing field (H) in the range of -10000 to 10000 Oe (equivalent

to -797700 to 797700 A $m^{-1}$ in the SI) at room temperature. The magnetic saturation value is that value of M that is reached when any subsequent increase in H does not cause an increase in the magnetization of the material.

***Determination of total fixed and volatile particles in suspension***

In general in this study, the determination of the particles in suspension was performed by means of the method described in standard UNE-EN 872 for the determination of particles in suspension using vacuum filtration. In that sense, a known volume of the aqueous matrix to be analyzed was filtered using an apparatus under a vacuum or low pressure through a glass fiber filter of known weight. Subsequently, the filter containing the retained particles was dried at 105°C, and the particle mass was determined by means of a weight difference with respect to the original filter. In the described examples, vacuum filtration was performed using a microporous membrane filter with a pore size of 0.45 μm. Next, the filter with the solids was dried in a drying oven for 2 h at 105°C. Once dry and cold, the filter containing the solids was weighed on a precision scale, and the mass of total solids ($ST_M$) was determined by the weight difference with respect to the same filter. For determining the fixed solid particles in suspension (inorganic, $SI_M$) and volatile solid particles in suspension (organic, $SO_M$), the filter containing the solid particles that have been retained, dried at 105°C is subjected to burning at 550°C for at least one hour and until reaching a constant mass value. Once cold, the filter containing the solids was weighed on a precision scale and the mass of total solids fixed solid particles in suspension was determined by the weight difference with respect to the same filter. The mass of volatile solid particles in suspension was determined by the difference between the mass of total solids and the mass of volatile solids.

In the performed oxidation experiments, the extent of the removal of the organic solid particles present in the different samples was calculated. In that sense, the percentage of removal of the organic solid particles in suspension was calculated from the difference between the mass of solid particles in suspension contained in the sample to be oxidized ($SS_{initial}$) and the mass of solid particles in suspension determined after the oxidation process ($SS_{final}$) (Equation 8).

$$Removal\ (\%) = \frac{SS_{initial} - SS_{final}}{SS_{initial}}\ x\ 100 \qquad (8)$$

***Example 1. Effect of the presence of other non-plastic organic solid particles on the separation of polystyrene microplastics in synthetic aqueous matrices***

Polystyrene microplastic separation experiments were carried out using aqueous matrices to which non-plastic organic solid particles having a size similar to microplastics were added. A magnetic iron mineral, i.e., ground magnetite (with a mean particle diameter of 0.2 μm under dry conditions and a particle size distribution as shown in Figure 1), was used to perform the separation process. The crystalline character of the magnetite used was confirmed by means of x-ray diffraction analysis, which also showed that the only phase present in the solid is magnetite ($Fe_3O_4$). The percentage of iron of the magnetic mineral used was in the range of 68 - 78% by weight. In turn, the magnetic saturation value of the material was in the range of 70 to 90 emu/g, which corresponds with $70 \cdot 10^3$ to $90 \cdot 10^3$ A/m in SI units.

The experiments were performed in glass vials in which the aqueous matrix, the microplastics, the non-plastic organic solid particles, and the magnetite particles were introduced. The solid particles (microplastics, non-plastic organic solid particles, and magnetite particles) remained in suspension in the aqueous matrices. A conductivity meter and a pH-meter were used to measure the conductivity and pH value, respectively. All the tests were performed with an aqueous matrix with a salt (sodium chloride) concentration of 0.1 g/L and pH value 7. All the experiments were performed in triplicate.

The volume of aqueous matrix used was 50 mL, and the amount of polystyrene microplastics added was 10 mg. In each test, there were added to said suspension 10 mg of one of the selected types of non-plastic organic solid particles with the same size range as that of the microplastics (all the solids were sifted in a size range of 100 to 250 μm using sieves with said mesh opening diameters) and 20 mg of magnetic iron mineral particles. The nature of the non-plastic solid particles added was changed by using organic particles (flour, crustacean exoskeleton, bivalve shell, pine needles, elm leaves, mimosa leaves, and cellulose). Next, the suspension was mechanically stirred for 30 s using a paddle stirrer. Subsequently, a magnetic field generated with a magnet was applied to separate the magnetic solids (free magnetic iron mineral and aggregates formed between the magnetic iron mineral and the microplastics or the non-plastic organic solid particles) from the aqueous matrix. Table 1 includes the studied microplastic and magnetic mineral sizes, the amounts of magnetite and microplastic added, the nature and the amounts of non-plastic organic solid particles in suspension added, and the yield achieved in the separation, both with respect to the separation of microplastics and with respect to the separation of non-plastic organic solid particles in suspension.

Table 1

| PS microplastics | | Magnetite | | Particles in suspension | | Separation yield (%) | |
|---|---|---|---|---|---|---|---|
| Size (μm) | Amount (mg) | Size (μm) | Amount (mg) | Nature | Amount (mg) | PS microplastic s | Solids in suspension |
| 100 - 250 | 10 | 0.2 | 20 | Flour | 10 | ≥99.0 | 70.0 ± 2.1 |
| | | | | Crustacean exoskeleton | | ≥99.0 | 95.2 ± 1.6 |
| | | | | Pine needles | | ≥99.0 | ≥99.0 |
| | | | | Elm leaves | | ≥99.0 | ≥99.0 |
| | | | | Mimosa leaves | | ≥99.0 | ≥99.0 |
| | | | | Cellulose | | ≥99.0 | ≥99.0 |

The separation of the polystyrene microplastics was very efficient, achieving recovery yields greater than 99.0%. It should be pointed out that no differences in the separation yields of polystyrene microplastics due to the presence of other non-plastic organic solid particles in suspension in the aqueous matrix were observed. However, the yields in the separation of said particles did in fact differ considerably based on their nature. In that sense, some, such as flour, were separated with lower yields, whereas crustacean exoskeleton, pine needles, elm leaves, and mimosa leaves, as well as cellulose were separated with a yield similar to that of microplastics. These results confirm that, regardless of the content of non-plastic organic solid particles in suspension of the water to be analyzed, the microplastics will be effectively separated from said matrix. However, it is very possible for the non-plastic organic particles in suspension to also be magnetically extracted from the matrix with a high yield, which will require subsequent oxidation to isolate the microplastics.

The leaching of iron coming from the magnetic iron mineral during the separation experiments was virtually negligible (<0.1 mg/L) regardless of the solids in suspension added to the aqueous matrix.

***Example 2. Application of the microwave-assisted Fenton process for the selective oxidation of the non-plastic organic solid particles magnetically separated together with microplastics from synthetic aqueous matrices***

Oxidation experiments for non-plastic organic solid particles were carried out after the experiment for the magnetic separation of said particles in the presence of polystyrene microplastics, as shown in Example 1. The magnetic separation of the solid particles was carried out by adding 50 mg of non-plastic organic solid particles (ground pine needles, sifted in a size range of 100 to 250 μm using sieves with said mesh opening diameters), 10 mg of polystyrene microplastic (100 - 250 μm), and 60 mg of magnetic iron mineral in a volume of 50 mL of water. The magnetic iron mineral used in the process of magnetic separation was ground magnetite with a mean particle diameter of 0.2 μm under dry conditions and a particle size distribution as shown in Figure 1. The crystalline character of the magnetite used was confirmed by means of x-ray diffraction analysis, which also showed that the only phase present in the solid is magnetite ($Fe_3O_4$). The percentage of iron of the magnetic mineral used was in the range of 68 - 78% by weight. In turn, the magnetic saturation value of the material was in the range of 70 to 90 emu/g, which corresponds with $70\cdot10^3$ to $90\cdot10^3$ A/m in SI units.

Next, the suspension was mechanically stirred for 1 min using a paddle stirrer which gave rise to the formation of magnetic iron mineral/microplastic aggregates and magnetic iron mineral/non-plastic organic solid particle aggregates. Subsequently, a magnetic field generated with a magnet was applied to magnetically separate the formed aggregates and the excess magnetic iron mineral from the aqueous matrix.

The magnetic separation yield (by aggregate formation), both for non-plastic organic solid particles (pine needles) and for polystyrene microplastics, was above 99.0%. The magnetically separated fraction was then subjected to the microwave-assisted Fenton oxidation process for the purpose of selectively removing non-plastic organic solid particles and thus being able to quantify the microplastic concentration.

Oxidation experiments for non-plastic organic solid particles present in the separated magnetic fraction were carried out by resuspending said fraction in a 50-mL volume of aqueous matrix. Oxidation reactions were carried out in glass Erlenmeyer flasks in which the aqueous matrix and the previously separated magnetic fraction (formed by magnetite/microplastic aggregates, magnetite/non-plastic organic solid particle aggregates and free magnetite) were introduced.

The solids forming the separated magnetic fraction in the previous step remained in suspension in the aqueous

matrices. The pH of the aqueous matrix was adjusted to 3 using nitric acid. A pH-meter was used to measure the pH value. All the experiments were performed in triplicate. Iron in solution (1 mL of a concentrated solution prepared using iron nitrate nonahydrate ($Fe(NO_3)_3$ * 9 $H_2O$) such that a final concentration of 10 mg/L in the reaction medium was reached) was added to said suspension as a catalyst, also adding hydrogen peroxide (1 mL of a concentrated solution to reach a final concentration in the medium of 5 g/L). Once the reagents were added, the Erlenmeyer flasks were immediately introduced in a microwave (Teka, MW 225G model, with a frequency of 2450 MHz) using a power of 700 W, corresponding to 55% of the maximum power of the microwave. Every 2 min, the flask was taken out of the microwave and the reagents (iron and hydrogen peroxide) were added again, repeating the process up to a total of five consecutive cycles.

Once the oxidation experiments ended, the content of solid particles in suspension was determined, and the removal yield for organic solid particles was calculated by the difference with respect to the amount of solids initially present (added microplastics, non-plastic organic solid particles, and magnetite) (Equation 1).

Table 2 includes the results obtained in the removal of non-plastic organic solid particles in suspension in the presence of polystyrene microplastics.

Table 2

| Solids in suspension | PS microplastics | Oxidation yield (%) | | |
|---|---|---|---|---|
| Nature | Amount (mg) | Amount (mg) | Non-plastic particles in suspension | PS microplastics |
| Pine needles | 50 | 10 | 89.5 ± 3.7 | 0.3 ± 0.1 |

Oxidation of the non-plastic organic solid particles by means of the microwave-assisted Fenton process with the addition of iron and hydrogen peroxide in five cycles lasting 2 min in duration was very efficient in the presence of magnetite, achieving removal yields for said particles of about 90%. It is also important to highlight that even though the polystyrene microplastics had the same size as the non-plastic organic solid particles, polystyrene microplastics were not affected by the oxidation treatment, with their mass being virtually intact at the end of the reaction. Furthermore, the composition and the size of the microplastics did not sustain any significant change after the treatment either. These results clearly show the suitability of the oxidation process developed for isolating microplastics in aqueous matrices which contain other non-plastic organic solid particles in suspension, even when they form part of the aggregates with magnetite.

***Example 3. Application of the microwave-assisted Fenton process for the selective oxidation of the non-plastic organic solid particles magnetically separated together with microplastics from real aqueous matrices***

Oxidation experiments for non-plastic organic solid particles present in real aqueous matrices were carried out after the experiment for the magnetic separation of said solids in the presence of polystyrene microplastics. 100 mg of polystyrene microplastic (100 - 250 $\mu$m) and 400 mg of magnetic iron mineral in a volume of 1 L were used for each of the real aqueous matrices evaluated. The following were used as aqueous matrices: river water, the secondary treatment effluent, and the tertiary treatment effluent from a wastewater treatment plant. A magnetic iron mineral, i.e., ground magnetite (with a mean particle diameter of 0.2 $\mu$m under dry conditions and a particle size distribution as shown in Figure 1) was used to perform the separation process. The crystalline character of the magnetite used was confirmed by means of x-ray diffraction analysis, which also showed that the only phase present in the solid is magnetite ($Fe_3O_4$). The percentage of iron of the magnetic mineral used was in the range of 68 - 78% by weight. In turn, the magnetic saturation value of the material was in the range of 70 to 90 emu/g, which corresponds with $70 \cdot 10^3$ to $90 \cdot 10^3$ A/m in SI units.

Next, the suspension was mechanically stirred for 30 s using a paddle stirrer which gave rise to the formation of magnetic iron mineral/microplastic aggregates and magnetic iron mineral/non-plastic organic solid particle aggregates. Subsequently, a magnetic field generated with a magnet was applied to magnetically separate the formed aggregates and the excess magnetic iron mineral from the aqueous matrix.

The yield achieved in magnetic separation (by aggregate formation) both for non-plastic organic solid particles present in the real aqueous matrix and for incorporated polystyrene microplastics was above 95.0%. The magnetically separated fraction was then subjected to the microwave-assisted Fenton oxidation process for the purpose of selectively removing the organic solids and thus being able to quantify the microplastic concentration.

Oxidation experiments for non-plastic organic solid particles present in the separated magnetic fraction were carried out by resuspending said fraction in a 50-mL volume of aqueous matrix. Oxidation reactions were carried out in glass Erlenmeyer flasks in which the aqueous matrix and the previously separated magnetic fraction (formed by magnetite/microplastic aggregates, magnetite/non-plastic organic solid particle aggregates and free magnetite) were introduced.

The solids forming the separated magnetic fraction in the previous step remained in suspension in the aqueous matrices. The pH of the aqueous matrix was adjusted to 3 using nitric acid. A pH-meter was used to measure the pH value. All the experiments were performed in triplicate. Iron in solution (1 mL of a concentrated solution prepared using iron nitrate nonahydrate ($Fe(NO_3)_3$ * 9 $H_2O$) such that a final concentration of 10 mg/L in the reaction medium was reached) was added to said suspension as a catalyst, also adding hydrogen peroxide (1 mL of a concentrated solution to reach a final concentration in the medium of 5 g/L). Once the reagents were added, the Erlenmeyer flasks were immediately introduced in a microwave (Teka, MW 225G model, with a frequency of 2450 MHz) using a power of 700 W, corresponding to 55% of the maximum power of the microwave. Every 2 min, the flask was taken out of the microwave and the reagents (iron and hydrogen peroxide) were added again, repeating the process up to a total of five consecutive cycles.

Once the oxidation experiments ended, the content of solids in suspension was determined, and the removal yield for organic solid particles was calculated by the difference with respect to the amount of solids initially present in the aqueous matrix (added microplastics and magnetite, together with the non-plastic organic solid particles) (Equation 8).

Table 3 includes the results obtained in the removal of non-plastic organic particles in the presence and absence of polystyrene microplastics.

Table 3

| Real aqueous matrices | | | | | Oxidation yield (%) | |
|---|---|---|---|---|---|---|
| Nature | Total solids (mg/L) | Percentage of volatile solids (%) | Total organic carbon (mg/L) | Conductivity (μS/cm) | Non-plastic particles in suspension | PS microplastics |
| River water | 59.7 | 100 | 22.4 | 660 | 83.1 ± 4.1 | 0.2 ± 0.1 |
| Secondary effluent | 8.7 | 100 | 9.2 | 881 | 85.8 ± 5.9 | 0.4 ± 0.2 |
| Tertiary effluent | 12.0 | 100 | 7.9 | 488 | 85.3 ± 5.7 | 0.3 ± 0.1 |

Oxidation of the non-plastic organic solid particles present in real aqueous matrices of a different nature by means of the microwave-assisted Fenton process with the addition of iron and hydrogen peroxide in five cycles lasting 2 min in duration was very efficient in the presence of magnetite, achieving removal yields for said particles greater than 80%. It is also important to highlight that the polystyrene microplastics were not affected by the oxidation treatment, with their mass being virtually intact at the end of the reaction. Furthermore, the nature and the size of the microplastics did not sustain significant changes after treatment either. These results clearly show the suitability of the oxidation process developed for isolating microplastics in aqueous matrices which contain non-plastic organic solid particles in suspension, even when they form part of the aggregates with magnetite.

***Example 4. Quantification of the microplastics present in a synthetic aqueous matrix containing organic (non-plastic) and inorganic particles using quantification method 1***

The quantification of the microplastics present in a synthetic aqueous matrix using quantification method 1 was carried out using a synthetic aqueous matrix in which non-plastic organic and inorganic solid particles were added. A magnetic iron mineral, i.e., ground magnetite (with a mean particle diameter of 0.2 μm under dry conditions and a particle size distribution as shown in Figure 1) was used to perform the separation process. The crystalline character of the magnetite used was confirmed by means of x-ray diffraction analysis, which also showed that the only phase present in the solid is magnetite ($Fe_3O_4$). The percentage of iron of the magnetic mineral used was in the range of 68 - 78% by weight. In turn, the magnetic saturation value of the material was in the range of 70 to 90 emu/g, which corresponds with $70 \cdot 10^3$ to $90 \cdot 10^3$ A/m in SI units.

The experiments were performed in glass vials in which the aqueous matrix, the microplastics, the non-plastic organic solid particles, the inorganic solid particles, and the magnetite particles were introduced. The solid particles (microplastics, non-plastic organic solid particles, inorganic particles, and magnetite particles) remained in suspension in the aqueous matrices. A conductivity meter and a pH-meter were used to measure the conductivity and pH value, respectively. All the tests were performed with an aqueous matrix with a salt (sodium chloride) concentration of 0.1 g/L and pH value 7. All the experiments were performed in triplicate.

The volume of aqueous matrix used was 50 mL and the amount of polystyrene microplastics added was 20 mg. There were added to said suspension 50 mg of non-plastic organic solid particles (pine needles) and 20 mg of

inorganic solid particles (sand), both selected with the same size range as that of the microplastics (all the solids were sifted in a size range of 100 to 250 μm using sieves with said mesh opening diameters), and 50 mg of magnetic iron mineral particles. Next, the suspension was mechanically stirred for 30 s using a paddle stirrer. Subsequently, a magnetic field generated with a magnet was applied to separate the magnetic solids (free magnetic iron mineral and aggregates formed between the magnetic iron mineral and the microplastics or the non-plastic organic solid particles or inorganic particles) from the aqueous matrix. The solids magnetically separated from the aqueous matrix were filtered and dried at 105°C for at least 2 h. Once dry and cold, the filter containing the solids was weighed on a precision scale, and the mass of solids was determined by the weight difference with respect to the same filter, obtaining parameter A.

The magnetically separated fraction (A) was then subjected to the microwave-assisted Fenton oxidation process for the purpose of selectively removing the organic solids and thus being able to quantify the microplastic concentration.

Oxidation experiments for non-plastic organic solid particles present in the separated magnetic fraction were carried out by resuspending said fraction in a 50-mL volume of aqueous matrix. Oxidation reactions were carried out in glass Erlenmeyer flasks in which the aqueous matrix and the previously separated magnetic fraction (formed by aggregates and free magnetite) were introduced.

The solids forming the separated magnetic fraction in the previous step remained in suspension in the aqueous matrices. The pH of the aqueous matrix was adjusted to 3 using nitric acid. A pH-meter was used to measure the pH value. Iron in solution (1 mL of a concentrated solution prepared using iron nitrate nonahydrate ($Fe(NO_3)_3 * 9 H_2O$) (concentration of the salt in the concentrated solution: 3618 mg/L) such that a final concentration of 10 mg/L in the reaction medium was reached) was added to said suspension as a catalyst, also adding hydrogen peroxide (1 mL of a concentrated solution to reach a final concentration in the medium of 5 g/L). Once the reagents were added, the Erlenmeyer flasks were immediately introduced in a microwave (Teka, MW 225G model, with a frequency of 2450 MHz) using a power of 700 W, corresponding to 55% of the maximum power of the microwave. Every 2 min, the flask was taken out of the microwave and the reagents (1 mL of the concentrated solution of iron and 1 mL of the hydrogen peroxide solution) were added again, repeating the process up to a total of five consecutive cycles.

Once the oxidation experiments ended, a magnetic field generated with a magnet was applied to separate the magnetic solids from the aqueous matrix. The solids magnetically separated from the aqueous matrix were filtered and dried at 105°C for at least 2 h. Once dry and cold, the filter containing the solids was weighed on a precision scale, and the mass of solids was determined by the weight difference with respect to the same filter, obtaining parameter AF. Next, the same filter was subjected to burning at 550°C for at least one hour and until reaching a constant mass value. Once cold, the filter containing the solids was weighed on a precision scale, and the mass of solids was determined by the weight difference with respect to the same filter, obtaining parameter AFM.

The mass of non-plastic organic solid particles that was indeed subjected to the microwave-assisted Fenton oxidation process, parameter $MO_{np,a}$, was determined by means of equation 2:

$$MO_{np,a} = (A - AF) * \frac{1}{a} \qquad \text{Equation 2}$$

in which a is 0.8.

The weight of microplastics ($MP_a$) contained in the sample was finally determined by means of equation 1:

$$MP_a = A - AFM - MO_{np,a} \qquad \text{Equation 1}$$

An example calculation for one of the three replicates performed is shown below:
Parameters A, AF, and AFM obtained were 107.7, 75.8, and 48.3 mg, respectively. These parameters served as the basis for calculating $MO_{np,a}$:

$$MO_{np,a} = (A - AF) * \frac{1}{a} = (107{,}7 - 75{,}8) * \frac{1}{0.8} = 39.9\,mg$$

The mass of the microplastics of the sample ($MP_a$) was finally determined:

$$MP_a = A - AFM - MO_{np,a} = 107.7 - 48.3 - 39.9 = 19.5\,mg$$

Table 4 includes mean values of the parameters obtained and of the microplastics quantified in the sample.

| Parameter | | Mass (mg) |
|---|---|---|
| Experimentally measured | A | 112 ± 3.8 |
| | AF | 80.7 ± 4.3 |
| | AFM | 48.7 ± 0.9 |
| Calculated | $MO_{np,a}$ | 39.2 ± 0.8 |
| | $MP_a$ | 24.1 ± 4.1 |

***Example 5. Quantification of the microplastics present in a synthetic aqueous matrix containing organic (non-plastic) and inorganic particles using quantification method 2***

The quantification of the microplastics present in a synthetic aqueous matrix using quantification method 2 was carried out using a synthetic aqueous matrix in which non-plastic organic and inorganic solid particles were added. A magnetic iron mineral, i.e., ground magnetite (with a mean particle diameter of 0.2 μm under dry conditions and a particle size distribution as shown in Figure 1) was used to perform the separation process. The crystalline character of the magnetite used was confirmed by means of x-ray diffraction analysis, which also showed that the only phase present in the solid is magnetite ($Fe_3O_4$). The percentage of magnetic iron mineral used was in the range of 68 - 78% by weight. In turn, the magnetic saturation value of the material was in the range of 70 to 90 emu/g, which corresponds with $70 \cdot 10^3$ to $90 \cdot 10^3$ A/m in SI units.

The experiments were performed in glass vials in which the aqueous matrix, the microplastics, the non-plastic organic solid particles, the inorganic solid particles, and the magnetite particles were introduced. The solid particles (microplastics, non-plastic organic solid particles, inorganic particles, and magnetite particles) remained in suspension in the aqueous matrices. A conductivity meter and a pH-meter were used to measure the conductivity and pH value, respectively. All the tests were performed with an aqueous matrix with a salt (sodium chloride) concentration of 0.1 g/L and pH value 7. All the experiments were performed in triplicate.

The volume of aqueous matrix used was 50 mL and the amount of polystyrene microplastics added was 20 mg. There were added to said suspension 50 mg of non-plastic organic solid particles (pine needles) and 20 mg of inorganic solid particles (sand), both selected with the same size range as that of the microplastics (all the solids were sifted in a size range of 100 to 250 μm using sieves with said mesh opening diameters), and 50 mg of magnetic iron mineral particles. Next, the suspension was mechanically stirred for 30 s using a paddle stirrer.

A control sample was also prepared with a 50-mL volume of aqueous matrix, in which exactly the same amounts of polystyrene microplastic, non-plastic organic solid particles (pine needles), and (inorganic) solid particles, were added under the same conditions described above, but without the addition of magnetic iron mineral. Said sample also the suspension was mechanically stirred for 30 s using a paddle stirrer. The concentration of total solids in suspension ($ST_M$), as well as the concentration of fixed solids in suspension ($SI_M$) in this control sample were determined. The concentration of volatile solids ($SO_M$) can be calculated by the difference between total solids ($ST_M$) and fixed solids ($SI_M$).

Next, a new aqueous matrix in a volume of 50 mL was prepared to which the same amounts of polystyrene microplastics, non-plastic organic solid particles, inorganic solid particles, and magnetic iron mineral (M) were added. Said matrix was stirred for 30 s using a paddle stirrer.

Subsequently, a magnetic field generated with a magnet was applied to separate the magnetic solids (free magnetic iron mineral and aggregates formed between the magnetic iron mineral and the microplastics or the non-plastic organic solid particles or inorganic particles) from the aqueous matrix (STA), and also collecting the supernatant.

This supernatant was collected in a filter that is dried at 105°C for 2 h. Once dry and cold, the filter containing the solids of the supernatant was weighed on a precision scale, and the mass of solids of the supernatant was determined by the weight difference with respect to the same filter, obtaining parameter $ST_{SN}$.

Next, the same filter was subjected to burning at 550°C for at least one hour and until reaching a constant mass value. Once cold, the filter containing the solids was weighed on a precision scale, and the mass of total solids ($ST_{SN}$) and inorganic solids ($SI_{SN}$) of the supernatant was determined by the weight difference with respect to the same filter before being subjected to burning. The concentration of volatile solids of the supernatant ($SO_{SN}$) can be calculated by the difference between total solids ($ST_{SN}$) and inorganic solids ($SI_{SN}$).

The magnetically separated fraction (STA) was then subjected to the microwave-assisted Fenton oxidation process for the purpose of selectively removing the organic solids and thus being able to quantify the microplastic concentration.

Oxidation experiments for non-plastic organic solid particles present in the separated magnetic fraction were carried out by resuspending said fraction in a 50-mL volume of aqueous matrix. Oxidation reactions were carried out in glass Erlenmeyer flasks in which the aqueous matrix and the previously separated magnetic fraction (formed by aggregates and free magnetite) were introduced using the reaction conditions described in Example 4.

After the Fenton process, the effluent was collected in a filter and dried at 105°C for 2 h. Once dry and cold, the filter containing the total solids of the aggregate that have not been oxidized was weighed on a precision scale, and the mass of total solids of the aggregate that have not been oxidized was determined by the weight difference with respect to the same filter, obtaining parameter $STNOX_A$.

Next, the same filter was subjected to burning at 550°C for at least one hour and until reaching a constant mass value. Once cold, the filter containing the solids was weighed on a precision scale, and the mass of inorganic solids of the aggregate was determined by the weight difference with respect to the same filter before being subjected to burning obtaining parameter $SI_A$.

Next, the total organic solids of the aggregate before being oxidized in Fenton, $SO_A$, were calculated:

$$SO_A = \left( (SI_{SN} + SI_A - M) \cdot \frac{ST_M - SI_M}{SI_M} \right) - (ST_{SN} - SI_{SN}) \quad \text{Equation 7}$$

After that, the aggregate weight loss in terms of organic matter, PPSO, was calculated:

$$PPSO = \frac{(SO_A + SI_A - STNOX_A)}{SO_A} \cdot 100 \quad \text{Equation 6}$$

Next, the non-plastic organic matter existing in the sample, RMPSO, was obtained:

$$RMPSO = (1-(PPSO/a)) / (PPSO/a) \quad \text{Equation 5}$$

wherein a is 0.8.

After that, the percentage of microplastics contained in the aggregate was obtained:

$$\%MP = (1-(1 / (RMPSO + 1) )) * 100 \quad \text{Equation 4}$$

The weight of microplastics ($MP_a$) contained in the sample was finally determined by means of equation 3:

$$MP_M = (\%MP \cdot SO_A)/100 \quad \text{Equation 3}$$

An example calculation performed for one of the three replicates is shown below:
The amount of magnetic mineral introduced was 50.1 mg and the volume of aqueous matrix used was 50 mL.

Parameters $SI_{SN}$, $ST_{SN}$, $STNOX_A$, and $SI_A$ obtained were 16.83 mg, 21.5 mg, 76.8 mg, and 48.83 mg, respectively. The concentration of total solids in suspension ($ST_M$), as well as the concentration of fixed solids in suspension ($SI_M$) were determined in the control sample, being 90.8 and 20.3 mg, respectively. These parameters served as the basis for calculating parameters $SONO_A$, $SO_A$, $ST_A$, $SOOX_A$, $PPSO_A$, RMPSO, and %MPa:

$$SO_A = \left( (SI_{SN} + SI_A - M) \cdot \frac{ST_M - SI_M}{SI_M} \right) - (ST_{SN} - SI_{SN}) = \left( (16.83 + 48.83 - 50.1) \cdot \frac{90.8 - 20.3}{20.3} \right) - (21.5 - 16.83) = 49.368\,mg$$

$$PPSO = \frac{(SO_A + SI_A - STNOX_A)}{SO_A} \cdot 100 = \frac{(49.368 + 48.83 - 76.8)}{49.368} \cdot 100 = 43.344\%$$

$$RMPSO = (1-(PPSO/a \cdot 100)) / (PPSO/a \cdot 100) = (1-(43.15/80))/(43.15/80) = 0.845$$

$$\%MP = (1-(1 / (RMPSO + 1) )) * 100= (1-(1/(0.85+1)))*100= 45.8\%$$

Lastly, the mass of microplastics in the sample ($MP_a$) was determined:

$$MP_M = (\%MP \cdot (SO_A)) \cdot 100 = \frac{45.8 \cdot 49.368}{100} = 22.61\, mg$$

## Claims

1. A process for determining parameters for the quantification of microplastics in an aqueous matrix comprising microplastics and non-plastic organic particles, and optionally inorganic particles, the process comprising:

a1) providing a first sample of the aqueous matrix comprising microplastics, non-plastic organic particles, and optionally inorganic particles,
b1) adding magnetic iron material particles to the aqueous matrix provided in step a1) to form microplastic/magnetic iron material particle aggregates and to form non-plastic organic particle/magnetic iron material particle aggregates,
c1) separating the formed aggregates and the free magnetic material particles from the mixture of step b1) by applying a magnetic field,
d1) subjecting the aggregates and the free magnetic iron material particles separated in step c1) to a step of drying,
e1) determining the weight of dry solids of step d1), obtaining parameter A,
f1) preparing an aqueous suspension of the aggregates and the free magnetic iron material particles obtained in step d1),
g1) subjecting the aqueous suspension prepared in step f1) to a microwave-assisted Fenton process,
h1) separating the aggregates and the free magnetic material particles present in the aqueous suspension resulting from step g1), preferably by means of filtration,
i1) subjecting the aggregates and the free magnetic iron material particles separated in step h1) to a step of drying,
j1) determining the weight of dry solids in step i1), obtaining parameter AF,
k1) subjecting the aggregates and the free magnetic material particles separated in step i1) to a step of combustion, and
l1) determining the weight of solids obtained after step k1), obtaining parameter AFM.

2. A process for determining parameters for the quantification of microplastics in an aqueous matrix comprising microplastics and non-plastic organic particles, and optionally inorganic particles, the process comprising:

a2) providing a first sample of the aqueous matrix comprising microplastics, non-plastic organic particles, and optionally inorganic particles,
b2) separating the solids from the sample of step a2), preferably by means of filtration,
c2) subjecting the solids separated in step b2) to a step of drying,
d2) determining the weight of dry solids of step c2), obtaining parameter $ST_M$,
e2) subjecting the dry solids of step c2) to a step of combustion, and
f2) determining the weight of solids obtained after step e2), obtaining parameter $SI_M$,
g2) providing a second sample of the aqueous matrix comprising microplastics, non-plastic organic particles, and optionally inorganic particles,
h2) adding magnetic iron material particles to the aqueous matrix provided in step g2) to form microplastic/magnetic iron material particle aggregates and to form non-plastic organic particle/magnetic iron material particle aggregates,
i2) separating the formed aggregates and the free magnetic material particles from the mixture of step h2) by applying a magnetic field,
j2) preparing an aqueous suspension of the aggregates and the free magnetic iron material particles separated in step i2),
k2) subjecting the aqueous suspension prepared in step j2) to a microwave-assisted Fenton process,
l2) separating the aggregates and the free magnetic material particles present in the aqueous suspension resulting from step k2), preferably by means of filtration,
m2) subjecting the aggregates and the free magnetic iron material particles separated in step 12) to a step of drying,
n2) determining the weight of dry solids in step m2), obtaining parameter $STNOX_A$,
o2) subjecting the aggregates and the free magnetic material particles separated in step m2) to a step of combustion,
p2) determining the weight of solids obtained after step o2), obtaining parameter $SI_A$,
q2) filtering the residual aqueous fraction obtained after the separation of step i2),
r2) subjecting the solids separated in step q2) to a step of drying,
s2) determining the weight of dry solids of step r2), obtaining parameter $ST_{SN}$, and
t2) subjecting the dry solids of step r2) to a step of combustion,
u2) determining the weight of solids obtained after step t2), obtaining parameter $SI_{SN}$.

3. The process according to claim 1 or 2, wherein the weight of the magnetic iron material particles which is added to the aqueous matrix in steps b1) or h2) is 0.1 to 20 times the weight of particles in suspension of the aqueous matrix provided in steps a1) or g2), respectively.

4. The process according to any of the preceding claims, wherein the magnetic iron material comprises an iron species selected from the group consisting of Fe(II), Fe(III), metallic Fe, and a mixture thereof, preferably the magnetic iron material is magnetite.

5. The process according to any of the preceding claims, wherein the average diameter of the magnetic iron mineral particles is comprised between 0.05 and 10 $\mu$m, preferably between 0.1 and 0.3 $\mu$m.

6. The process according to any of the preceding claims, wherein the microplastics are selected from the group consisting of polystyrenes (PS), polyethylene terephthalates (PET), polyethylenes (PE) including high-density polyethylenes (HDPE) and low-density polyethylenes (LDPE), polyvinyl chlorides (PVC), polypropylenes (PP), polycarbonates (PC) and polyurethanes (PU), and mixtures thereof.

7. The process according to any of the preceding claims, wherein the mixture obtained in steps b1) or/and h2) are subjected to stirring before performing steps c1) or/and i2), respectively, preferably wherein the stirring is mechanical or magnetic stirring, more preferably mechanical stirring.

8. The process according to any of the preceding embodiments, wherein the separation of steps c1) and/or i2) is performed using magnets or electromagnets.

9. The process according to any of the preceding claims, wherein in steps g1) or k2) hydrogen peroxide and an iron salt are added, preferably hydrogen peroxide and an iron salt selected from the group consisting of iron nitrate, iron chloride, iron sulfate, and mixtures thereof, more preferably hydrogen peroxide and iron nitrate.

10. The process according to claim 9, wherein the iron salt concentration in the aqueous suspension of steps g1) or k2) is in the range of 1 mg/L to 1000 mg/L and/or the hydrogen peroxide concentration in the aqueous suspension of steps g1) or k2), respectively, is in the range of 10 mg/L to 100 g/L.

11. The process according to any of the preceding claims, wherein step g1) or k2) is performed with a microwave power in the range of 50 to 1500 W and/or for a time in the range of 1 s to 2 h.

12. The process according to any of the preceding claims, wherein the pH of the aqueous suspension of steps g1) and/or k2) is adjusted in the range of 2 to 4.

13. The process according to any of the preceding claims, wherein the combustion of steps k1), e2), o2), and/or t2) is performed by subjecting the solids to a temperature of between 500°C and 600°C for 1 to 4 hours.

14. A process for the quantification of microplastics in an aqueous matrix comprising microplastics and non-plastic organic particles, and optionally inorganic particles, the process comprising:

I-1) performing steps a1)-l1) of the process for determining parameters according to claims 1 and 3-13 for determining parameters A, AF, and AFM;
II-1) calculating the weight of microplastics ($MP_A$) by means of equation 1:

$$MP_A = A - AFM - MO_{NP,A} \quad \text{Equation 1}$$

wherein

A is the weight of solids determined in step e1) of the process according to claims 1 and 3-13,
AFM is the weight of solids determined in step l1) of according to claims 1 and 3-13, $MO_{NP,A}$ is calculated by means of equation 2

$$MO_{NP,A} = (A - AF) * \frac{1}{a} \quad \text{Equation 2}$$

wherein A is the weight of solids determined in step e1) of the process according to claims 1 and 3-13, AF is the weight of solids determined in step j1) of the process according to claims 1 and 3-13, and a is a

number between 0.7 and 1, preferably between 0.8 and 0.9, most preferably 0.8.

**15.** A process for the quantification of microplastics in an aqueous matrix comprising microplastics and non-plastic organic particles, and optionally inorganic particles, the process comprising:

I-2) performing steps a2)-u2) of the process for determining parameters according to claims 2-13 for determining parameters $ST_M$, $SI_M$, $STNOX_A$, $SI_A$, $ST_{SN}$, and $SI_{SN}$;
II-2) calculating the weight of microplastics ($MP_M$) by means of equation 3:

$$MP_M = (\%MP \cdot (SO_A))/100 \qquad \text{Equation 3}$$

wherein

%MP is calculated by means of equation 4

$$\%MP = \left(1 - \left(\frac{1}{RMPSO+1}\right)\right) \cdot 100 \qquad \text{Equation 4}$$

wherein RMPSO is calculated by means of equation 5

$$RMPSO = \left(1 - \left(\frac{PPSO}{a \cdot 100}\right)\right) / \left(\frac{PPSO}{a \cdot 100}\right) \qquad \text{Equation 5}$$

wherein a is a number between 0.7 and 1, preferably between 0.8 and 0.9, most preferably 0.8, and PPSO is calculated by means of equation 6

$$PPSO = \frac{(SO_A + SI_A - STNOX_A)}{SO_A} \cdot 100 \qquad \text{Equation 6}$$

wherein $STNOX_A$ is the weight of solids determined in step n2) of the process according to claims 2-13, $SI_A$ is the weight of solids determined in step p2) of the process according to claims 2-13, and $SO_A$ is calculated by means of equation 7

$$SO_A = \left((SI_{SN} + SI_A - M) \cdot \frac{ST_M - SI_M}{SI_M}\right) - (ST_{SN} - SI_{SN}) \qquad \text{Equation 7}$$

wherein $SI_A$ is the weight of solids determined in step p2) of the process according to claims 2-13, $SI_{SN}$ is the weight of solids determined in step u2) of the process according to claims 2-13, $ST_{SN}$ is the weight of solids determined in step s2) of the process according to claims 2-13, M is the weight of magnetic material particles added in step h2) of the process according to claims 2-13, $ST_M$ is the weight of solids determined in step d2) of the process according to claims 2-13, and $SI_M$ is the weight of solids determined in step f2) of the process according to claims 2-13.

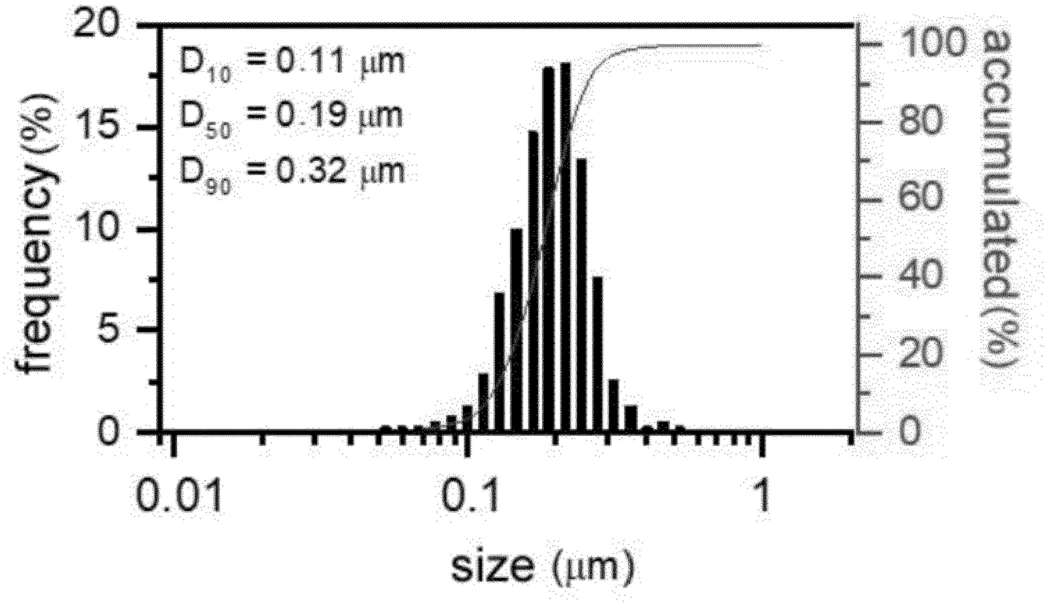
D10 = 0.11 μm
D50 = 0.19 μm
D90 = 0.32 μm
frequency (%)
accumulated (%)
size (μm)
20
15
10
5
0
100
80
60
40
20
0
0.01
0.1
1

FIG. 1

Europäisches Patentamt
European Patent Office
Office européen des brevets

# EUROPEAN SEARCH REPORT

Application Number
EP 22 38 2061

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 2020/240069 A1 (UNIV MADRID AUTONOMA [ES]) 3 December 2020 (2020-12-03)<br>* page 19, lines 20-33 *<br>* page 20, lines 8-12 *<br>----- | 1,3-13 | INV.<br>C02F1/48<br>G01N1/34<br>G01N1/44<br>G01N5/04<br>G01N33/18 |
| Y | MALLOW OLE ET AL: "A new thermoanalytical method for the quantification of microplastics in industrial wastewater", ENVIRONMENTAL POLLUTION, BARKING, GB, vol. 259, 20 December 2019 (2019-12-20), XP086090667, ISSN: 0269-7491, DOI: 10.1016/J.ENVPOL.2019.113862 [retrieved on 2019-12-20]<br>* section 2.5 *<br>----- | 1,3-13 | |
| A | YANG Y ET AL: "Microwave enhanced Fenton-like process for the treatment of high concentration pharmaceutical wastewater", JOURNAL OF HAZARDOUS MATERIALS, ELSEVIER, AMSTERDAM, NL, vol. 168, no. 1, 30 August 2009 (2009-08-30), pages 238-245, XP026161961, ISSN: 0304-3894, DOI: 10.1016/J.JHAZMAT.2009.02.038 [retrieved on 2009-02-20]<br>* the whole document *<br>-----<br>-/-- | 1,9-12 | TECHNICAL FIELDS SEARCHED (IPC)<br>C02F<br>G01N |

The present search report has been drawn up for all claims

1

| Place of search | Date of completion of the search | Examiner |
|---|---|---|
| The Hague | 15 June 2022 | Böhler, Robert |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

page 1 of 2

Europäisches Patentamt
European Patent Office
Office européen des brevets

# EUROPEAN SEARCH REPORT

Application Number
EP 22 38 2061

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | XU X-Y ET AL: "Microplastic ingestion reduces energy intake in the clamAtactodea striata", MARINE POLLUTION BULLETIN, vol. 124, no. 2, 20 February 2009 (2009-02-20), pages 798-802, XP085259254, ISSN: 0025-326X, DOI: 10.1016/J.MARPOLBUL.2016.12.027 * section 2.4 * ----- | 2,14,15 | |

TECHNICAL FIELDS SEARCHED (IPC)

The present search report has been drawn up for all claims

1

| Place of search | Date of completion of the search | Examiner |
|---|---|---|
| The Hague | 15 June 2022 | Böhler, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 22 38 2061

This annex lists the patent family members relating to the patent documents cited in the above-mentioned European search report. The members are as contained in the European Patent Office EDP file on
The European Patent Office is in no way liable for these particulars which are merely given for the purpose of information.

15-06-2022

| Patent document cited in search report | Publication date | Patent family member(s) | Publication date |
|---|---|---|---|
| WO 2020240069 A1 | 03-12-2020 | EP 3978446 A1 | 06-04-2022 |
| | | ES 2796998 A1 | 30-11-2020 |
| | | WO 2020240069 A1 | 03-12-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- ES 2796998 A1, Munoz, M.
- WO 2020240069 A1


### Non-patent literature cited in the description


- **MACARTHUR, D.E. et al.** The New Plastics Economy: Rethinking the Future of Plastics. *World Economic Forum,* 2016
- **BERGMANN, M. et al.** Marine Anthropogenic Litter. Springer, 2015
- **GRBIC, J. et al.** *Environmental Science and Technology Letters,* 2019, vol. 6, 68-72
- **COLE, M. et al.** *Nature Scientific Reports,* 2014, vol. 4 (4528), 1-8
- **WIECZOREK, A.M. et al.** *Environmental Science and Technology,* 2019, vol. 53, 5387-5396
- **WRIGHT, S.L. et al.** *Environmental Science and Technology,* 2017, vol. 51, 6634-6647
- **COX, K.D. et al.** *Environmental Science and Technology,* 2019, vol. 53, 7068-7074
- **LI, J. et al.** *Water Research,* 2018, vol. 137, 362-374
- **KOELMANS, A.A. et al.** *Water Research,* 2019, vol. 155, 410-422
- **DRIS, R. et al.** *Environmental Chemistry,* 2015, vol. 12, 592-599
- Microplastics in drinking-water. World Health Organization, 2019
- **PRATA, J.C. et al.** *Trends in Analytical Chemistry,* 2019, vol. 110, 150-159
- **STOCK, F. et al.** *Trends in Analytical Chemistry,* 2019, vol. 113, 84-92
- **FELISING, S. et al.** *Environmental Pollution,* 2018, vol. 234, 20-28
- **SGIER, L. et al.** *Nature Communication,* 2016, vol. 7, 11587
- **CLAESSENS, M. et al.** *Marine Pollution Bulletin,* 2013, vol. 70, 227-233
- **HURLEY, R.R. et al.** *Environmental Science and Technology,* 2018, vol. 52, 7409-7417
- **LÖDER, M.G. et al.** *Environ. Sci. Technol.,* 2017, vol. 51, 14283-14292
- **TAGG, A.S. et al.** *Chem. Commun.,* 2017, vol. 53, 372-375
- **MUNNO, K. et al.** *Environ. Toxicol. Chem.,* 2018, vol. 37, 91-98
- **HURLEY, R.R. et al.** *Environ. Sci. Technol.,* 2018, vol. 52, 7409-7417
- **CUNSOLO, S. et al.** *Anal. Bioanal. Chem.,* 2021, vol. 413, 3789-3799